# EUROPÄISCHE PATENTSCHRIFT

(11) **EP 4 552 660 B1**
(45) Veröffentlichungstag und Bekanntmachung des Hinweises auf die Patenterteilung: **08.10.2025**
(21) Anmeldenummer: 23208246.1
(22) Anmeldetag: 07.11.2023
(51) Int. Cl.: A61L 27/50, B82Y 40/00

(54) **VERFAHREN ZUR ERZEUGUNG EINER ABRIEBFESTEN NANOSTRUKTUR, TRÄGERKÖRPER MIT EINER SOLCHEN NANOSTRUKTUR SOWIE ZUGEHÖRIGE VERWENDUNG**
METHOD FOR PRODUCING AN ABRASION-RESISTANT NANOSTRUCTURE, SUPPORT BODY COMPRISING SUCH A NANOSTRUCTURE AND ASSOCIATED USE
PROCÉDÉ DE PRODUCTION D'UNE NANOSTRUCTURE RÉSISTANTE À L'ABRASION, CORPS DE SUPPORT DOTÉ D'UNE TELLE NANOSTRUCTURE ET UTILISATION ASSOCIÉE

(43) Veröffentlichungstag der Anmeldung: 14.05.2025
(73) Patentinhaber: Nanoshape GmbH, 76131 Karlsruhe (DE)
(72) Erfinder: DOLL, Patrick, 76131 Karlsruhe (DE)
(74) Vertreter: Mertzlufft-Paufler, Cornelius

(56) Entgegenhaltungen:
- ECOFFEY SERGE ET AL: "Technology platform for the fabrication of titanium nanostructures", JOURNAL OF VACUUM SCIENCE AND TECHNOLOGY: PART B, AVS / AIP, MELVILLE, NEW YORK, NY, US, vol. 29, no. 6, 1 November 2011 (2011-11-01), pages 6FG06 - 6FG06, XP012154959, ISSN: 1071-1023, [retrieved on 20111103], DOI: 10.1116/1.3657517
- MARTÍNEZ-CHÁVEZ L A ET AL: "Nanocomposite Bi/TiO2 multilayer thin films deposited by a crossed beam laser ablation configuration", APPLIED PHYSICS A, SPRINGER BERLIN HEIDELBERG, BERLIN/HEIDELBERG, vol. 127, no. 11, 3 October 2021 (2021-10-03), XP037618512, ISSN: 0947-8396, [retrieved on 20211003], DOI: 10.1007/S00339-021-04957-0

## Beschreibung

Die Erfindung betrifft ein Verfahren zum Erzeugen einer abriebfesten Nanostruktur auf einer Oberfläche, insbesondere auf Teilbereichen dieser Oberfläche, was als eine Art Nanobonding der Nanostruktur auf die Oberfläche verstanden werden kann. Diese Oberfläche kann durch einen Trägerkörper gebildet sein, wobei sich die Erfindung insbesondere dann vorteilhaft einsetzten lässt, wenn der Trägerkörper in Form eines dauerhaften oder temporären Implantats ausgestaltet ist. Bei dem Verfahren wird zunächst in einem ersten Prozessschritt A die Nanostruktur auf die Oberfläche aufgebracht, wobei die Nanostruktur insbesondere auf der Oberfläche aufgewachsen werden kann. Wird im Folgenden von Nanostruktur gesprochen, so sind damit stets mehrere nanoskopische Elemente bzw. mehrere Nanostrukturen gemeint.

Die Erfindung betrifft ferner einen Schichtverbund, der eine solche Nanostruktur umfasst, sowie einen Trägerkörper, der einen solchen Schichtverbund auf seiner Oberfläche trägt.

Schließlich betrifft die Erfindung auch bestimmte Verwendungen eines solchen Schichtverbunds zu spezifischen Zwecken.

### Hintergrund

Aktuelle Entwicklungen im Feld der Implantologie werden durch die Notwendigkeit vorangetrieben, eine Reihe von Herausforderungen im Bereich der medizinischen Implantate zu bewältigen.

Beispielsweise ist die Verlängerung der Lebensdauer von Implantaten ein wesentliches Ziel, welches der Patientensicherheit dient. Implantate sind verschiedenen mechanischen Belastungen und Reibungen im menschlichen Körper ausgesetzt. Im Laufe der Zeit kann diese Abnutzung zur Verschlechterung der Eigenschaften des Implantats, insbesondere an seiner Oberfläche, führen. Eine erhöhte Lebensdauer würde die Notwendigkeit eines Implantataustauschs verringern und dem jeweiligen Patienten somit unnötige chirurgische Eingriffe ersparen.

Eine verbesserte Biokompatibilität ist ein weiteres wichtiges Ziel in der Weiterentwicklung von Implantaten. Dies ist wünschenswert, um Entzündungen, Allergien oder Abstoßungsreaktionen des Körpers zu vermeiden. Schließlich sind auch weitere Entwicklungen von Interesse, insbesondere wenn damit bakteriell-bedingte Implantat-Infektionen vermieden oder zumindest reduziert werden können.

Solche Eigenschaften von Implantaten können beispielsweise durch eine Nanostrukturierung der Oberfläche des Implantats realisiert werden. Überall dort, wo Nanostrukturen auf einer Trägeroberfläche, beispielsweise eines Implantats, aufgebracht werden sollen, besteht allerdings grundsätzlich die Herausforderung darin, eine ausreichende Haftung der Nanostruktur auf der Oberfläche zu erzielen. Oftmals wurde im Stand der Technik beobachtet, dass zwar Nanostrukturen herstellbar sind, diese aber bereits durch bloße Berührung von der jeweiligen Oberfläche wieder entfernt werden, sodass dann die von der Nanostruktur gelieferten Effekte wieder verloren gehen. Um also die von der Nanostruktur gelieferten technischen Effekte auch tatsächlich in praktischen Anwendungen dauerhaft nutzen zu können, ist es notwendig, die jeweilige Nanostruktur abriebfest auf der Oberfläche aufzubringen.

S. Ecoffey et al., J. Vac. Sci. Technol. B 29/6, 2011 beschreibt einen zweistufigen Prozess zur Herstellung von topdown Titannanostrukturen. Der erste Prozessschritt umfasst Elektronenstrahllithographie, gefolgt von einem Titan-Plasmaätzprozess. Der zweite Prozessschritt ist ein Damascene-Prozess, bei dem die Titannanostrukturen in das Oxid eingebettet werden.

L. A. Martinez-Chavez, Applied Physics A, 127:808, 2021 beschreibt ein Verfahren zur Herstellung von Bi/TiO2/Bi/TiO2/Bi/TiO2-Mehrschichtdünnfilmen mittels gepulster Laserabscheidung (PLD) unter Verwendung einer gekreuzten Strahlkonfiguration. Um die Bi-Schichten auf TiO2 zu stapeln, werden zwei Laserstrahlen verwendet, um so abwechselnd zwei senkrecht zueinander positionierte Targets (TiO2 und Bi) abzutragen.

Hiervon ausgehend liegt der vorliegenden Erfindung daher die Aufgabe zugrunde, eine abriebfeste Nanostruktur zur Verfügung zu stellen, die insbesondere für die Nutzung auf Implantatoberflächen geeignet ist.

### Zusammenfassung der Erfindung

Zur Lösung dieser Aufgabe wird ein Verfahren gemäß Anspruch 1 vorgeschlagen. Insbesondere wird somit bei einem Verfahren zum Erzeugen einer abriebfesten Nanostruktur wie eingangs beschrieben vorgeschlagen, dass die im Prozessschritt A erzeugte Nanostruktur in einem zweiten Prozessschritt B mittels einer Einbettschicht dauerhaft und abriebfest an die Oberfläche angebunden wird, wobei die Einbettschicht an der Oberfläche erzeugt wird und die Nanostruktur nur teilweise einbettet. Insbesondere kann dabei die Einbettschicht auf der Nanostruktur abgeschieden werden.

Die Abscheidung der Einbettschicht kann hierbei insbesondere vermittelt durch eine chemische Umwandlung einer obersten Werkstoffschicht des Trägerkörpers erfolgen, d.h. die Einbettschicht kann durch chemische Umwandlung einer obersten Werkstoffschicht des Trägerkörpers, insbesondere unter Volumenzunahme, erzeugt werden.

Die Erfindung beschreibt somit ein Verfahren zum Erzeugen einer Nanostruktur, die abriebfest mit der Oberfläche eines Trägerkörpers verbunden ist, was insbesondere im Bereich der Implantologie von großem Interesse ist. Die möglichen Anwendungen der Erfindung sind dabei aber nicht auf Implantate beschränkt, sondern erfindungsgemäße abriebfeste Nanostrukturen können überall dort eingesetzt werden, wo Oberflächeneigenschaften eines Trägerkörpers gezielt und dauerhaft verändert werden sollen.

Der Prozessschritt A kann dabei als eine Form der Nanostrukturierung verstanden werden. Der Prozessschritt B kann hingegen als eine Form einer mechanischen Anbindung der Nanostruktur auf nanoskopischem Level an den Trägerkörper verstanden werden. Durch das erfindungsgemäße Verfahren wird also eine Nanostruktur auf einer Oberfläche erzeugt, die mittels der zusätzlichen Einbettschicht mechanisch stabil an die Oberfläche angebunden ist/wird. Insbesondere bei Ausgestaltung der abriebfesten Nanostruktur auf einer Implantatoberfläche können so reproduzierbare und konstante, also dauerhafte, Oberflächeneigenschaften gewährleistet werden.

Das erläuterte Verfahren lässt sich gemäß der Erfindung auf vielfältige Weise weiterbilden:
So kann eine innere Schichtdicke D1 der Einbettschicht, gemessen unterhalb der ursprünglichen Oberfläche des Trägerkörpers, mindestens 6 nm, bevorzugt mindestens 12 nm, besonders bevorzugt mindestens 18 nm betragen. Die Einbettschicht kann dabei während des zweiten Prozessschrittes B sowohl in die Oberfläche des Trägermaterials hinein wachsen, als auch aus der ursprünglichen Oberfläche heraus wachsen. Dadurch wird die Abriebfestigkeit der Schicht weiter erhöht, weil eine robuste nanomechanische Verankerung mittels der Einbettschicht erreicht wird.

Ferner ist es bevorzugt, wenn die jeweilige Nanostruktur konisch zulaufend ausgestaltet ist. In diesem Fall zeigt somit eine Spitze der Nanostruktur von der Oberfläche weg und ein jeweiliger Zwischenraum bzw. Abstand zwischen den einzelnen Nanostrukturen nimmt mit zunehmender Entfernung von der Oberfläche zu. Eine solche Form ist einerseits günstig, damit die Nanostruktur eine gewünschte Abstoßungswirkung auf Mikroorganismen wie beispielsweise Bakterien entfaltet. Und andererseits kann so eine sehr zuverlässige nanomechanische Verankerung mittels der Einbettschicht erzielt werden, da diese beim Aufwachsen die Zwischenräume (zeitlich zunehmend) auffüllen und damit die Nanostruktur auf der Oberfläche verankern kann.

Eine maximale Schichtdicke/Einbetthöhe D2 der Einbettschicht, gemessen oberhalb der ursprünglichen Oberfläche des Trägerkörpers, kann bevorzugt kleiner als eine durchschnittliche Höhe H der Nanostruktur, erneut gemessen oberhalb der ursprünglichen Oberfläche des Trägerkörpers, sein. Denn in diesem Fall ragt die Nanostruktur aus der Einbettschicht heraus. Hierbei ist es bevorzugt, wenn die Nanostruktur mit wenigstens 20%, bevorzugt mit wenigstens 50%, besonders bevorzugt mit wenigstens 66% der durchschnittlichen Höhe H aus der Einbettschicht herausragt. Das Herausragen der Nanostruktur führt dazu, dass oberflächenaktive Eigenschaften, beispielweise anti-adhäsive oder bakterizide Eigenschaften, der nanostrukturierten Oberfläche auch nach Erzeugung der Einbettschicht zumindest teilweise erhalten bleiben. Denn diese Eigenschaften werden durch die Nanostruktur verursacht, genauer durch die Morphologie der Nanostruktur.

Die Einbettschicht kann also, vorzugsweise mittels eines elektrochemischen Verfahrens und/oder mittels eines Oxidationsverfahrens, so abgeschieden (bzw. erzeugt) werden, dass die Einbettschicht sowohl in den Trägerkörper hineinwächst als auch aus der ursprünglichen Oberfläche herauswächst. Eine solche Prozessführung kann insbesondere derart ausgestaltet sein, dass nach Abschluss des Prozessschritts B die Einbettschicht tiefer in den Trägerkörper hinein reicht als die Nanostruktur, vorzugsweise sodass die Einbettschicht die Nanostruktur nicht nur einbettet, sondern auch im Trägerkörper nanoskopisch mechanisch verankert. Eine Einbettschicht, die in die Oberfläche hinein und auf der Oberfläche aufwächst, erhöht die mechanische Stabilität der Schicht, bzw. des Schichtsystems aus Nanostruktur und Einbettschicht, weil dadurch die Einbettschicht mit dem Trägerkörper auf nanoskopischer Ebene verwächst. Dadurch wird die Abriebfestigkeit der Nanostruktur auf dem Trägerkörper wesentlich erhöht.

Der erste Prozessschritt A kann beispielsweise eine hydrothermische Oxidation sein, die in einem Druckreaktor durchgeführt wird. Insbesondere kann während des Prozessschritts A die Nanostruktur nur auf der Oberfläche, insbesondere auf einer nativen Oxidschicht des Trägerkörpers, aufwachsen, nicht aber in den Trägerkörper hinein (wie die Einbettschicht). Die Oxidation kann vorzugsweise unter Verwendung von Reinstwasser oder von wässrigem Ammoniak oder von Kaliumhydroxid (KOH) als reaktiver Flüssigkeit vollzogen werden. Besonders bevorzugt ist ferner, wenn die Oberfläche des Trägerkörpers, auf welcher die Nanostruktur aufgebracht werden soll, während des Prozessschritts A vollständig in die reaktive Flüssigkeit eingetaucht bleibt.

Der Trägerkörper kann beispielsweise aus Metall, insbesondere aus Titan, gefertigt sein. In einem solchen Fall wird durch die hydrothermische Oxidation ein Metalloxid, insbesondere Titanoxid, in Form von Nanopartikeln entstehen, welches/die die Nanostruktur ausbildet/ausbilden. Die Nanostrukturierung durch hydrothermische Oxidation ist vorteilhaft, weil sie zu einer homogenen Oberflächenstrukturierung, insbesondere auch von komplex geformten Trägerstrukturen, führt. Damit ist das Verfahren insbesondere geeignet zur Strukturierung von Implantatoberflächen, die eine komplexe 3D-Geometrie aufweisen.

Der zweite Prozessschritt B kann zum Beispiel eine elektrochemische Anodisation zur Erzeugung der Einbettschicht sein. Diese Anodisation kann vorzugsweise unter Verwendung eines flüssigen, vorzugsweise Fluor-freien (also keine Fluor-haltige Verbindungen enthaltenden), Elektrolyten wie beispielsweise verdünnter Schwefelsäure oder vergleichbaren geeigneten Elektrolyten durchgeführt werden.

Ferner kann der Prozessschritt B unter Einsatz einer elektrischen Gleichspannung für die Anodisation durchgeführt werden. Hierbei ist es bevorzugt, wenn die Gleichspannung, unter 20 V, bevorzugt von unter 15 V, besonders bevorzugt unter 10 V liegt, weil sich dadurch verlässlich und gut steuerbar eine geeignete Schichtdicke der Einbettschicht einstellen lässt.

Die elektrochemische Anodisation kann beispielsweise mit einer Einwirkzeit von unter 30 min, bevorzugt von unter 5 min, besonders bevorzugt von unter 2 min, ausgeführt werden, wodurch sehr kurze Gesamtprozesszeiten möglich sind. Eine elektrochemische Anodisation der nanostrukturierten Oberfläche führt zu einer sehr homogenen Erzeugung der Einbettschicht, auch auf komplex geformten (Implantat-)Trägerkörpern. Dadurch kann eine konforme Oberflächenbeschichtung realisiert werden. Die Verwendung niedriger Spannungen und kurzer Prozessdauern macht das Verfahren dabei besonders einfach in der Anwendung sowie kostengünstig, da das Verfahren mit simplen Apparaturen durchführbar ist und einen hohen Durchsatz erlaubt. Zudem ist dieses Verfahren schonend anwendbar für komplex geformte Trägerkörper.

Alternativ hierzu kann der zweite Prozessschritt B auch eine nasschemische Oxidation zur Erzeugung der Einbettschicht sein, vorzugsweise unter Verwendung einer oxidierenden Flüssigkeit. Besonders bevorzugt ist es dabei, wenn die Oberfläche des Trägerkörpers, auf welcher die Einbettschicht abgeschieden werden soll, während des Prozessschritts B vollständig mit samt der Nanostruktur in die oxidierende Flüssigkeit eingetaucht bleibt. Eine nasschemische Oxidation zur Erzeugung der Einbettschicht erlaubt es, abriebfeste Nanostrukturen auf Trägerkörpern aus verschiedenen Materialen und mit verschiedenen Material- und Oberflächeneigenschaften zu erzeugen. Insbesondere kann HNO3, H2SO4, HCLO4, H2O2, KOH oder NaOH oder (wässrige) Zusammensetzungen hiervon als oxidierende Flüssigkeit bei der Oxidation verwendet werden.

Der zweite Prozessschritt B kann auch mittels eines plasmagestützten oder plasmainduzierten Verfahrens zur Erzeugung der Einbettschicht, vorzugsweise unter Verwendung eines Sauerstoff-haltigen Plasmas, realisiert werden. Dadurch können auch Oberflächen anderer Materialien unter Verwendung verschieden zusammengesetzter Plasmen durch chemisches und/oder physikalisches Einwirken homogen modifiziert werden.

Die mit dem Verfahren hergestellte Nanostruktur kann Nanopartikel aufweisen, deren Spitzen im Mittel gleichgerichtet, insbesondere normal, zu der Oberfläche ausgerichtet sind. Hierdurch lassen sich sehr homogene Oberflächeneigenschaften einstellen.

Vorzugweise können die Nanopartikel eine überwiegend tetragonal pyramidale und/oder tetragonal bi-pyramidale und/oder eine konische und/oder eine doppelkonische Grundform oder eine konische Grundform aufweisen. Die Nanopartikel können ferner eine durchschnittliche Höhe H, gemessen oberhalb der ursprünglichen Oberfläche, von weniger als 300 nm, bevorzugt von weniger als 250 nm, besonders bevorzugt von weniger als 175 nm, aufweisen.

Darüber hinaus können die Nanopartikel einen durchschnittlichen Spitzenabstand von weniger als 300 nm, bevorzugt von weniger als 250 nm, besonders bevorzugt von weniger als 150 nm, weiter besonders bevorzugt von weniger als 75 nm aufweisen. Liegen die Spitzenabstände hingegen bei größeren Werten, so wurde beobachtet, dass dann die gewünschte Abstoßungswirkung auf Bakterien nur noch in unzureichendem Maße erzielt werden kann. Solche Parameter sind vorteilhaft, um homogene Eigenschaften der Oberfläche zu erzeugen. Denn durch die gleichmäßige Beschaffenheit der Nanostrukturen kann eine homogene Oberflächenbeschaffenheit des behandelten Trägerkörpers gewährleistet werden. Die Nanostrukturen können dabei derart geformt sein, dass sie beispielsweise anti-adhäsive und/oder Bakterien-abstoßende und/oder bakterizide Eigenschaften der Oberfläche erzeugen. Insbesondere bakterizide Eigenschaften sind dabei erst bei den genannten Strukturgrößen der Nanopartikel (zumindest teilweise) zu beobachten.

In einer Ausführungsform ist der Trägerkörper aus Metall, vorzugsweise aus Titan oder aus einer Titanlegierung, gefertigt. In diesem Fall kann die in Prozessschritt B hergestellte Einbettschicht eine amorphe, teilamorphe oder rein kristalline Titanoxidschicht sein und/oder die in Prozessschritt A hergestellte Nanostruktur kann dann durch eine, zumindest teilweise, kristalline Titanoxidschicht gebildet sein. Bei einer solchen Materialwahl sind die thermischen und/oder elektrochemischen o.g. Prozesse besonders gut reproduzierbar durchführbar. Die Metalloberflächen können dabei, noch vor Anwendung des erfindungsgemäßen Verfahrens eine Oxidschicht, insbesondere eine natürliche Oxidschicht oder aber eine bereits zuvor gezielt erzeugte Oxidschicht, aufweisen.

In einer Ausführungsform weist die Oberfläche des Trägerkörpers entsprechend eine Oxidschicht, vorzugsweise eine Titanoxidschicht (z.B. TiO2 oder TiOx), auf, und die Nanostruktur wird auf diese Oxidschicht aufgebracht oder aus dieser Oxidschicht durch (morphologische) Umwandlung der Oxidschicht hervorgebracht. Auch in diesem Fall sind die thermischen und elektrochemischen Prozesse reproduzierbar durchführbar. Die Oxidschicht kann hierbei vorzugsweise eine natürliche Oxidschicht des Trägermaterials sein.

In einer Ausführungsform des Verfahrens kann eine natürliche und/oder zuvor zusätzlich erzeugte Oxidschicht durch einen vorangegangenen Prozess (z.B. Ätzen) zunächst entfernt werden.

Ein (auf der ursprünglichen Oberfläche des Trägerkörpers) aufgewachsener Anteil der Einbettschicht kann aufgrund der beschriebenen Prozessführung eine Struktur und/oder Form aufweisen, die komplementär zu einer Form der Nanostruktur ist. Die Einbettschicht, genauer deren aufgewachsener Anteil, kann auch Zwischenräume zwischen einzelnen Nanostrukturen (die auf der Oberfläche im Schritt A erzeugt wurden) zumindest teilweise ausfüllen. Dadurch kann, insbesondere bei konischer Ausgestaltung der Nanostrukturen (sodass diese also nach außen spitz zulaufen), eine nanomechanische Verkeilung zwischen Einbettschicht und Nanostruktur erreicht werden.

Beispielsweise kann der aufgewachsene Anteil der Einbettschicht Aussparungen aufweisen, die in ihrer Form der Grundfläche der Nanostruktur und (z.B. in Richtung der Normalen der Oberfläche folgend) komplementär zu einer, insbesondere konischen, Form/Gestalt der Nanostruktur sind. Mit anderen Worten kann die Einbettschicht derart ausgestaltet sein, dass ihr aufgewachsener Anteil, also der Anteil oberhalb der ursprünglichen Oberfläche, Zwischenräume zwischen Elementen der Nanostruktur (beispielsweise Zwischenräume zwischen den beschriebenen pyramidenförmigen Nanostrukturen) zumindest teilweise auffüllt.

Dieses Auffüllen bzw. Einbetten kann dabei durch geeignete Parameterwahl, insbesondere durch geeignete elektrische Spannung und/oder Dauer, des Prozessschritts B so eingestellt werden, dass die Einbettschicht Zwischenräume zwischen den Nanostrukturen bis zu einer bestimmten Einbetthöhe oberhalb der ursprünglichen Oberfläche auffüllt. Diese Einbetthöhe (oder "maximale Schichtdicke D2" der Einbettschicht gemessen oberhalb der ursprünglichen Oberfläche) entspricht somit der Höhe des aufgewachsenen Anteils der Einbettschicht (während die Gesamtdicke der Einbettschicht höher ausfallen kann, wenn es einen Anteil der Einbettschicht gibt, der in den Trägerkörper eingewachsen ist). Da die Nanostrukturen aus der Einbettschicht herausragen sollen, damit sie die gewünschten Oberflächeneffekte, insbesondere eine Abstoßungswirkung auf Bakterien, entfalten können, ist es bevorzugt, wenn die Einbetthöhe kleiner als eine maximale Höhe der Nanostrukturen (erneut gemessen oberhalb der ursprünglichen Oberfläche) ausfällt. Bevorzugt kann die Einbetthöhe höchstens halb so hoch wie eine maximale oder durchschnittliche Höhe der einzubettenden Nanostruktur gewählt sein.

Mit einer derart ausgestalteten Einbettschicht lassen sich somit insbesondere einzelne Elemente der Nanostruktur, insbesondere konisch zulaufende Elemente wie beispielsweise Pyramiden, von der Einbettschicht keilförmig einfassen. Dadurch kann eine zuverlässige nanomechanische Verankerung der Nanostruktur auf dem Trägerkörper erzielt werden.

Ein weiterer Aspekt der Erfindung beschreibt einen Schichtverbund umfassend eine Nanostruktur. Diese Nanostruktur kann wie erläutert abriebfest auf einer Oberfläche eines Trägerkörpers aufgebracht sein und/oder mittels eines Verfahrens wie zuvor erläutert und/oder nach einem der auf ein Verfahren gerichteten Ansprüche erzeugt, insbesondere auf dem Trägerkörper aufgebracht oder aufgewachsen, sein. Der Schichtverbund zeichnet sich nun dadurch aus, dass die Nanostruktur Nanopartikel aufweist, die wenigstens einen oberflächenaktiven Effekt, beispielsweise einen optischen Effekt und/oder einen anti-adhäsiven Effekt und/oder einen bakteriziden Effekt, erzeugen, und ferner dadurch, dass die Nanostruktur zumindest teilweise in eine Einbettschicht eingebettet ist. Bevorzugt steht dabei die Nanostruktur aus der Einbettschicht so weit hervor, dass wenigstens ein oberflächenaktiver Effekt zumindest teilweise erhalten bleibt. Mit anderen Worten müssen also nicht alle oberflächenaktiven Effekte zwingend nach dem Nanobonding noch erhalten sein.

Ein weiterer erfindungsgemäßer Aspekt beschreibt einen Schichtverbund aus einer solchen abriebfesten Nanostruktur und einer Einbettschicht. Die Schichtkomponenten können auf der Oberfläche eines Trägerkörpers, beispielsweise durch thermische, oxidative oder elektrochemische Verfahren, aufgebracht werden. Nanopartikel der Nanostruktur können dabei zu besonderen Eigenschaften der Oberfläche führen. Insbesondere beeinflussen solche Nanopartikel neben optischen Eigenschaften die Benetzbarkeit der Oberfläche und/oder erzeugen anti-adhäsive und/oder bakterizide Effekte. Die Nanopartikel sind bevorzugt in die Einbettschicht eingefasst, ragen aber so weit aus der Einbettschicht heraus, dass die durch sie erzeugten Oberflächeneigenschaften zumindest teilweise erhalten bleiben. Dadurch entsteht eine mechanisch stabile, reproduzierbar herstellbare Oberfläche mit gezielt einstellbaren Oberflächeneigenschaften.

Die Einbettschicht kann so weit über die Nanostruktur, insbesondere nach innen in Richtung des Trägerkörpers, hinausreichen, dass eine nanomechanische Verankerung der Nanostruktur auf einer Oberfläche des Trägerkörpers und/oder in dem Trägerkörper erzielt ist. D.h. die Einbettschicht kann insbesondere tiefer in den Trägerkörper hineinreichen im Vergleich zur/als die Nanostruktur. Durch ein Einwachsen der Einbettschicht in die Oberfläche des Trägerkörpers wird die Abriebfestigkeit des Schichtsystems aus Nanostruktur und Einbettschicht weiter erhöht. Mit anderen Worten kann die Einbettschicht in den Bulk des Trägerkörpers hineinreichen. In diesem Fall überschreitet die Einbettschicht die ursprüngliche Grenzfläche zwischen Trägerkörper und Nanostruktur, und zwar in einer Richtung, die weg zeigt von den außenseitigen Spitzen der Nanostrukturen.

Die Einbettschicht kann sich mindestens morphologisch von der Nanostruktur unterscheiden. Andererseits können die Einbettschicht und die Nanostruktur aus demselben Material, insbesondere aus einem Metalloxid, hergestellt sein. Dadurch wird die Nanostruktur abriebfest von der Einbettschicht eingefasst, ohne dass die Nanostruktur ihre oberflächenaktiven Eigenschaften und/oder ihre Morphologie / Kristallstruktur verliert.

Ein weiterer erfindungsgemäßer Aspekt beschreibt einen Trägerkörper, insbesondere in Form eines Implantats, mit einem wie oben beschriebenen Schichtverbund. Vorzugsweise kann sich auch hier die Einbettschicht morphologisch von der Nanostruktur unterscheiden und/oder die Einbettschicht kann zumindest teilweise in den Trägerkörper eingewachsen sein. Ein solcher Trägerkörper kann dauerhaft vorteilhafte Oberflächeneigenschaften aufweisen, die durch die Nanostruktur verursacht werden, weil letztere abriebfest von der Einbettschicht eingefasst und damit auf dem Trägerkörper mechanisch verankert ist, und zwar ohne, dass die Nanostruktur dabei ihre oberflächenaktiven Eigenschaften und/oder ihre gewünschte Morphologie und Kristallstruktur ändert oder verliert.

Ein weiterer Aspekt der Erfindung beschreibt eine spezifische Verwendung eines wie oben beschriebenen Schichtverbunds auf einem Trägerkörper zur Erzeugung maßgeschneiderter Oberflächeneigenschaften des Trägerkörpers. Hierbei kann der Schichtverbund mit einem wie oben beschriebenen Verfahren und/oder mit einem Verfahren gemäß einem der Verfahrensansprüche hergestellt worden sein und/oder mit einem solchen Verfahren auf einer Oberfläche des Trägerkörpers abriebfest verankert worden sein. Die Verankerung kann hierbei mit dem erfindungsgemäßen Ansatz insbesondere so stark ausgestaltet werden, dass bei ausreichendem Druck ein zugehöriger Kontaktkörper des Trägerkörpers, beispielsweise ein Knochen, an der Nanostruktur abgerieben wird und nicht die Nanostruktur an dem Kontaktkörper, wenn der Trägerkörper unter dem Druck mit samt dem Schichtverbund auf dem Kontaktkörper entlang gleitet.

Der Schichtverbund, genauer die Nanostruktur des Schichtverbunds, kann zum Beispiel eine (zumindest teilweise) bakterizide Wirkung und/oder einen anti-adhäsiven Effekt erzeugen und dadurch beispielsweise eine gefährliche Biofilmbildung verlangsamen. Dadurch kann der Schichtverbund beispielsweise auf Oberflächen von Bauteilen verwendet werden, die hygienischen Standards entsprechen sollen und/oder die besonderer Kontamination, beispielsweise durch häufige Kontakte, ausgesetzt sind.

In einer Ausführungsform ist der Trägerkörper ein Implantat und der Schichtverbund erzeugt eine (zumindest teilweise oder zeitweise) bakterizide Wirkung und/oder einen anti-adhäsiven Effekt in Bezug auf die äußerste Oberfläche des Implantats, die durch die Nanostruktur mit ausgebildet wird.

Nachstehend wird die Erfindung anhand mehrerer bevorzugter Ausführungsbeispiele näher beschrieben.

Es zeigt:
Fig. 1 einen schematischen Querschnitt einer Ausführungsform eines Schichtverbunds auf einem Trägerkörper;
Fig. 2 einen schematischen Querschnitt einer Ausführungsform eines eingebetteten Nanostruktur auf der Oberfläche eines Trägerkörpers;
Fig. 3 ein Flussdiagramm, welches eine Ausführungsform eines erfindungsgemäßen Verfahrens zum Erzeugen einer abriebfesten Nanostruktur auf einer Oberfläche eines Trägerkörpers beschreibt;
Fig. 4 eine schematische Skizze der anti-adhäsiven Eigenschaft der nanostrukturierten Oberfläche;
Fig. 5 eine schematische Darstellung eines Druckreaktors zur Herstellung von Nanostrukturen;
Fig. 6 eine schematische Darstellung des Prozesses zur Nanostrukturierung mittels hydrothermischer Oxidation;
Fig. 7 eine schematische Ausgestaltung der Nanostruktur nach unterschiedlicher Behandlungsdauer;
Fig. 8 eine schematische Darstellung des Prozesses zur Herstellung einer Einbettschicht;
Fig. 9 eine schematische Darstellung eines Aufbaus einer elektrochemisches Anodisation;
Fig. 10 eine schematische Darstellung des Ein- und Aufwachsens einer Einbettschicht auf die Oberfläche des Trägerkörpers;
Fig. 11 eine schematische Darstellung der Abhängigkeit der Schichtdicke der Einbettschicht von der Prozessspannung und
Fig. 12 illustriert die komplementäre Form einer erzeugten erfindungsgemäßen Einbettschicht, wenn man sich die Nanostruktur 40 weg denkt.

Bei der folgenden Beschreibung verschiedener Ausführungsformen der Erfindung erhalten in ihrer Funktion übereinstimmende Elemente auch bei abweichender Gestaltung oder Formgebung übereinstimmende Bezugszahlen.

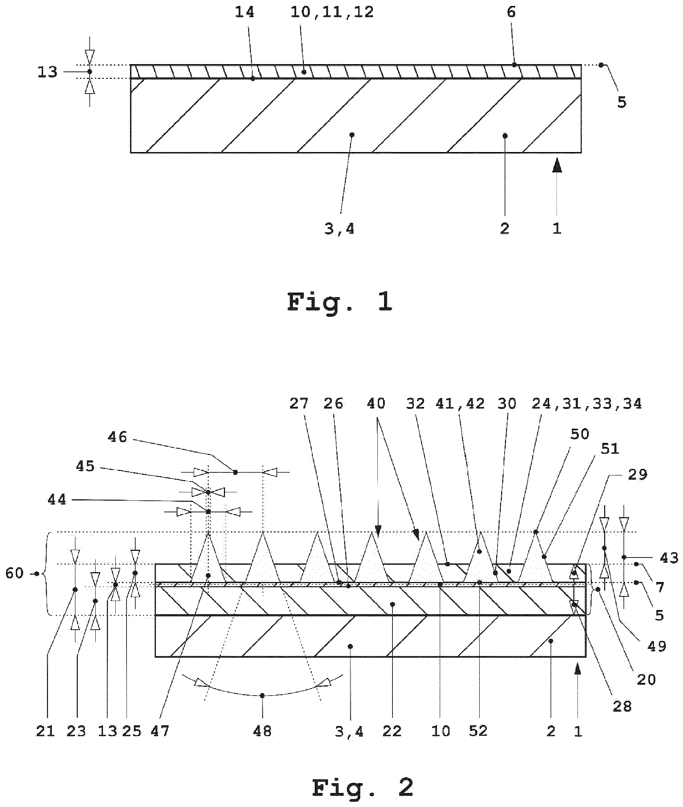

Figur 1 zeigt einen Querschnitt durch eine äußere Schicht, beispielsweise eines Implantats 1, umfassend einen Trägerkörper oder Grundkörper 2 und ggf. eine (insbesondere native) Oxidschicht 10.

Der Trägerkörper 2 kann vorzugsweise ein permanentes oder temporäres Implantat 1 sein. Der Trägerkörper 2 besteht bevorzugt aus einem Metall als Grundmaterial 3, insbesondere Titan, Edelstahl oder Aluminium, oder Metalllegierungen, bevorzugt aus Ti6Al4V, alternativ aus Chrom-Cobalt-Basislegierungen oder Aluminiumlegierungen. Dabei kann das Grundmaterial 3 ein Gefüge 4 aufweisen. Alternativ besteht der Trägerkörper 2 aus einer Keramik als Grundmaterial 3.

Bevorzugt ist der Trägerkörper 2 aus Titan oder einer Titanlegierung geformt und bildet eine native Oxidschicht 10 aus, die chemisch sehr inert ist und (zumindest teilweise) mechanisch bestandfähig, was die Ursache dafür ist, dass Titanimplantate im Knochen gut einwachsen können. Dies ist insbesondere bei der Endoprothetik aber auch bei zahnmedizinischen Implantaten vorteilhaft.

Die Oxidschicht 10 besteht aus einem Material 11, also beispielsweise dem natürlichen Oxid des Grundmaterials 3, im Falle von Titan oder Titanlegierungen aus TiO2 bzw. TiOx. Die natürliche Oxidschicht ist typischerweise 2-5 nm dick. Alternativ kann die Oxidschicht 10 auch eine zuvor aufgebrachte bzw. gezielt erzeugte Oxidschicht sein. Die Oxidschicht 10 kann beispielsweise durch chemische oder physikalische Depositionsverfahren, insbesondere PVD ("physical vapor deposition") oder CVD ("chemical vapor deposition")-Verfahren, Plasmaverfahren oder Sputterverfahren erzeugt worden sein. Die Oxidschicht weist ein Gefüge 12 auf und hat eine Schichtdicke 13. Zwischen der Oxidschicht 10 und dem Grundmaterial 3 kann eine Grenzfläche 14 bestehen.

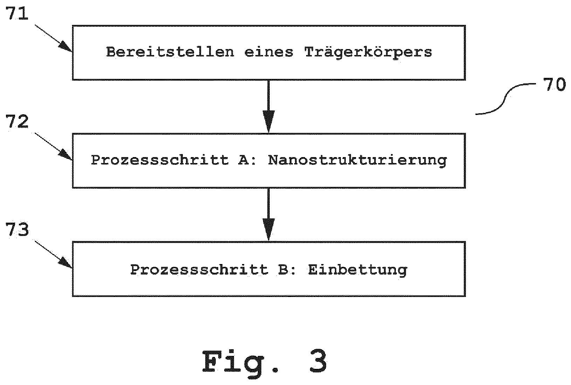

Die Oberfläche der Oxidschicht 10, also die Grenzfläche zwischen der Oxidschicht 10 und der Umgebung, beispielweise Luft, weist eine Oberflächenrauigkeit 6. Diese Grenzfläche wird im Folgenden auch als Ausgangsoberfläche 5 bezeichnet. Diese Oberfläche 5 bildet somit den Ausgangspunkt für das nachfolgend beschriebene erfindungsgemäße Verfahren, mit dem eine Nanostruktur 40 abriebfest auf der Oberfläche 5 verankert werden soll:
Das erfindungsgemäße Verfahren 70 zur Herstellung einer abriebfesten Nanostruktur 40 ist schematisch in Figur 3 beschrieben: Im ersten Schritt 71 wird ein Trägerkörper 3, etwa wie gerade beschrieben, bereitgestellt. In Schritt 72 wird im Prozessschritt A eine Nanostruktur auf der Oberfläche 5 erzeugt. Im Schritt 73 wird in Prozessschritt B eine Einbettschicht erzeugt. Die Prozessschritte A und B werden im Folgenden näher beschrieben.

In dem ersten Prozessschritt A wird eine Nanostruktur 40 auf dem Trägerkörper 2 erzeugt. Beispielsweise kann die Nanostruktur 40 gezielt auf bestimmten Teilen oder der gesamten Oberfläche 5 aufgebracht, aufgewachsen oder beispielsweise lithographisch auf diese Oberfläche 5 erzeugt werden. Die Maskierung von Teilbereichen der Oberfläche 5 kann hierbei bevorzugt mit positivem Photolack in Schichtdicken kleiner 10 µm, bevorzugt kleiner 2 µm durchgeführt werden. Die Nanostruktur 40 wird in diesem Prozessschritt A vorzugsweise vollständig und abschließend erzeugt. Das heißt, die Nanostruktur 40 wird durch nachfolgende Prozessschritte nicht oder nur unwesentlich in ihrer äußeren Form verändert.

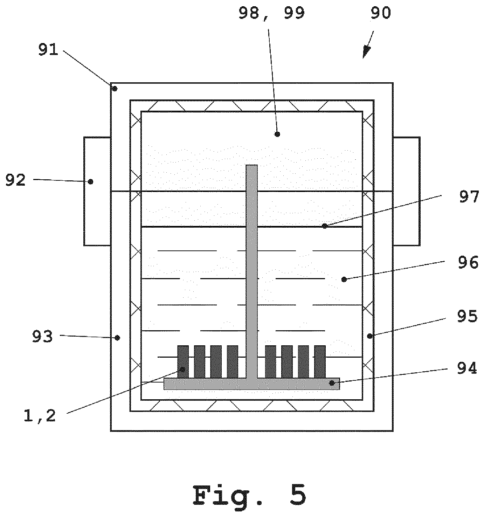

Bevorzugt wird die Nanostruktur 40 mittels einer hydrothermischen Oxidation erzeugt. Das Verfahren wird in einem Druckreaktor bei >15 bar, beispielweise bei 20 bar, Innendruck ausgeführt. Ein geeigneter Druckreaktor 90 ist in Figur 5 schematisch dargestellt. Der Druckreaktor 90 ist dabei mithilfe eines Edelstahlzylinders als Grundgefäß 93 ausgestaltet, der innenseitig mit Teflon (PTFE) 95 ausgekleidet ist.

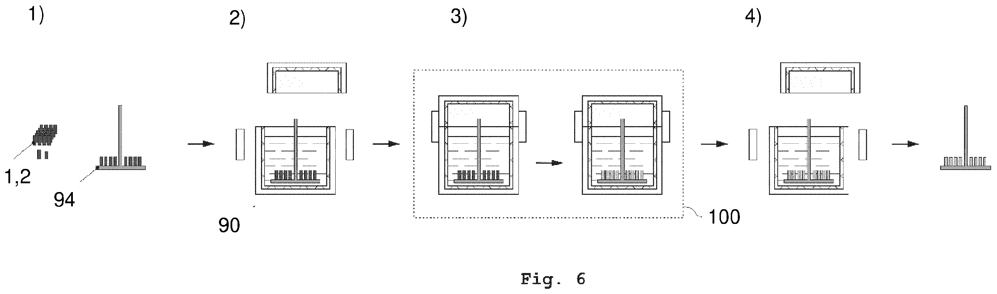

Der Prozess zur Nanostrukturierung der Oberfläche 5 des Trägerkörpers 2 ist schematisch in Figur 6 dargestellt. Die mit der Nanostruktur bzw. den Nanopartikeln 40 zu beschichtenden Trägerkörper 2, beispielsweise Titan-Implantate 1, werden dabei auf ein Teflon-Gestell 94 geladen (Schritt 1) in Fig. 6) und zusammen mit dem Gestell in den Druckreaktor 90 eingeführt (Schritt 2) in Fig. 6). Die Trägerkörper 2 können zuvor einem separaten Reinigungsschritt unterzogen werden. Die Trägerkörper 2 sind dabei vollständig in Reinstwasser 96 als verwendeter Reaktionslösung, wie anhand des Füllstands 97 gezeigt, eingetaucht. Bei einer Temperatur von 220 °C und einem Druck von ca. 20 bar wird dann über 24 Stunden die hydrothermische Oxidation durchgeführt, wobei dieser Prozess parallel an mehreren Implantaten 1 bzw. Trägerkörpern 2 durchgeführt werden kann (Schritt 3) in Fig. 6). Das Aufheizen kann in einem Ofen oder mittels einer Mantelheizung erfolgen. Nach Ablauf des Prozesses wird der Druckreaktor 90 entladen (Schritt 4) in Fig. 6).

Durch diesen Prozess wird eine nanostrukturierte kristalline Titanoxidschicht auf dem Titan-Implantat 1 aufgewachsen, wobei die Oxidfront dabei nicht in das Material hinein wächst, sondern gleichmäßig überall und in allen Raumrichtungen auf der Oberfläche 5 nach außen hin aufwächst. Dadurch kann eine sehr konforme Schichtdicke der Nanostruktur 40 erreicht werden und es ist keine Abschattung zu beobachten, sodass beispielsweise auch Hinterschnitte, scharfe Kanten oder Löcher mit der Nanostruktur 40 versehen werden können.

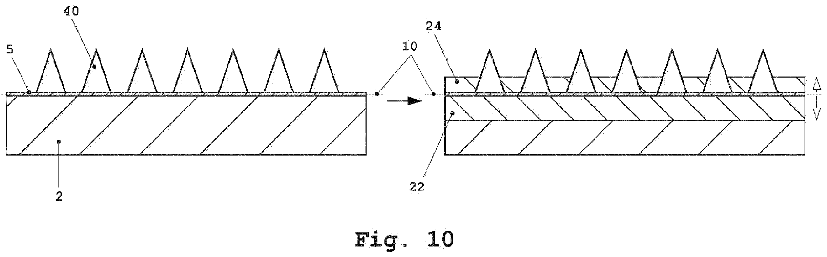

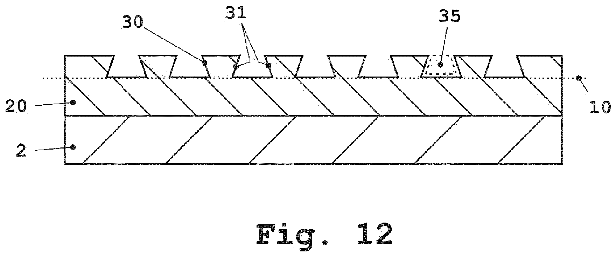

Figur 2 illustriert schematisch das Ergebnis des Verfahrens, nämlich eine mittels einer erfindungsgemäßen amorphen Einbettschicht 20 nur teilweise eingebettete aber dadurch abriebfest auf dem Trägerkörper 2 verankerte pyramidenförmige Nanostruktur 40, die konisch zuläuft (wobei die Spitze der Pyramide / des Konus von der Ausgangsoberfläche 5 weg zeigt). Man erkennt dabei anhand der Schraffur in Figur 2, dass die Einbettschicht 20 nach innen über die ursprüngliche Ausgangsoberfläche 5 hinaus in den Trägerkörper 2 eingewachsen ist (= eingewachsener Anteil 22). Ferner weist die Einbettschicht 20 aber auch einen aufgewachsenen Anteil 24 oberhalb der Ausgangsoberfläche 5 auf mit einer Einbetthöhe 103, die weniger als 50% einer maximalen Höhe 102 der pyramidenförmigen Nanostrukturen 40 beträgt (Vgl. dazu auch die Doppelpfeile in der rechten Teilabbildung der Figur 10). Der aufgewachsene Anteil 24 füllt dabei Zwischenräume zwischen den Nanostrukturen 40 aus und (sofern man sich die Nanostrukturen 40 wegdenkt) weist entsprechend Lücken an den Stellen der Nanostrukturen 40 auf, was das "Einbetten" herausstellt, wie in Figur 12 dargestellt. Mehr noch: Die Form des aufgewachsene Anteils 24 in den Zwischenräumen ist gerade komplementär zu der Pyramidenform der Nanostrukturen 40, die eine hohe Kristallinität aufweisen. Durch diese komplementäre Form 35 werden die Nanostrukturen 40 mit der Einbettschicht 20 regelrecht verkeilt und damit auf nanomechanisch fest am Trägerkörper 2 verankert. Diese Verankerung beruht somit auf einem geometrischen Effekt auf nanoskopischem Level.

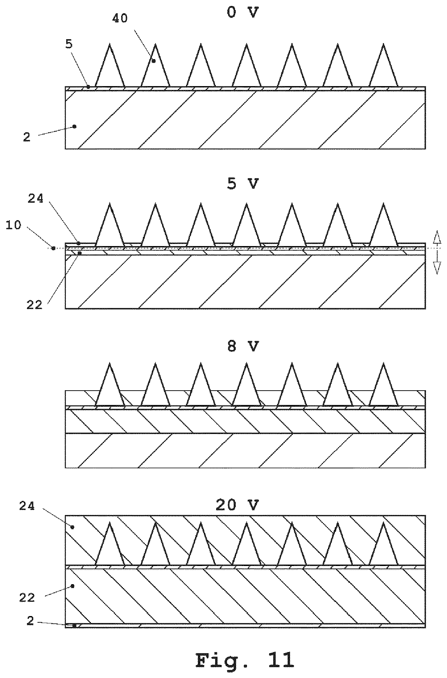

Dieses selektive Einbetten (welches man auch gut anhand der Figur 11 nachvollziehen kann - man beachte, dass die Form der Nanostruktur 40 unverändert beibehalten bleibt) wird im Prozessschritt B erzielt, durch welchen die chemisch unedle Einbettschicht 20 selektiv auf der (chemisch stabileren) Nanostruktur 40 abgeschieden wird, ohne dass dabei die kristalline Nanostruktur 40 in ihrer Form oder ihrer Morphologie maßgeblich verändert wird. Ursächlich hierfür ist die hohe Ordnung in der Kristallstruktur der Nanostruktur 40 im Vergleich zur überwiegend amorphen Struktur der Einbettschicht 20, was dazu führt, dass die Nanostrukturen 40 im Prozessschritt B (also z.B. durch eine elektrische Anodisation) aufgrund ihrer hohen chemischen Stabilität nicht oder nur geringfügig verändert werden, während die amorphe Einbettschicht 20 selektiv aufwächst. Eine solche Prozessführung gelingt beispielsweise besonders gut, wenn die Nanostrukturen 40 in Form von TiO2-Kristallen (z.B. Anatas) ausgebildet werden, wie in Figur 11 schematisch illustriert.

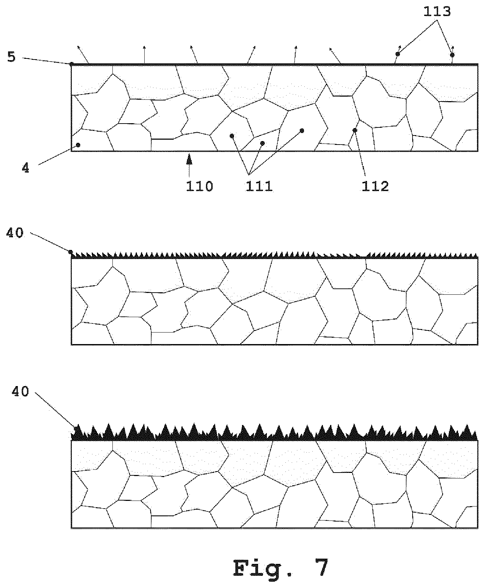

Figur 7 zeigt schematisch die Ausgestaltung der Nanostruktur 40 nach einer Behandlungsdauer der Trägerkörper 2 von weniger als 1 Stunde (Fig. 7, links) bei einer Temperatur von 220 °C und von 24 Stunden (Fig. 7, rechts) bei einer Temperatur von 220 °C. Die Korngröße des Gefüges der amorphen Titanoxidschicht 42 und/oder das Gefüge und die räumliche Ausrichtung der Körner der unterliegenden Werkstoffschicht (bulk) bestimmen dabei die Ausformung von Nanopartikeln. Die Nanopartikel wachsen anfänglich, beispielsweise nach 1 Stunde, in unterschiedlichen Raumrichtungen auf und richten sich aber nach einigen Stunden, beispielsweise 24 Stunden, Prozessdauer normal zur Oberfläche 5 aus. Die Nanopartikel zeigen somit eine im Mittel gleich gerichtete Ausrichtung, wie auch in Figur 2 mit der Normalenrichtung 47 markiert. Wenn entsprechende Prozessparameter gewählt sind, kann das Wachstum sehr gleichmäßig erfolgen, sodass dann pyramidenförmige Nanopartikel mit einem Öffnungswinkel 48 aufwachsen. Die Nanopartikel 40 haben dabei eine durchschnittliche Höhe 43, eine durchschnittliche Breite 44, eine durchschnittliche Spitzengröße 45 und einen durchschnittlichen Abstand zueinander 46.

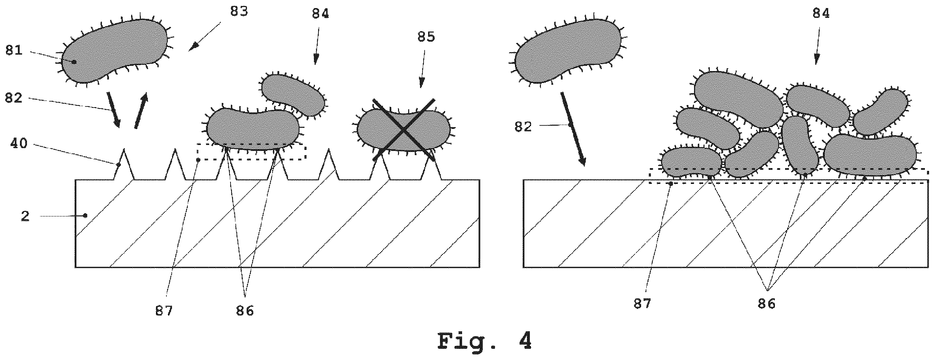

Figur 4 zeigt eine schematische Skizze zur Veranschaulichung der erwünschten anti-adhäsiven Eigenschaft der nanostrukturierten Oberfläche im Vergleich zu einer unstrukturierten Oberfläche. Die Nanostruktur 40 bewirkt, dass Bakterien 81 (in Figur 4 nierenförmig dargestellt) durch weniger Oberflächenkontakte 86 nicht bzw. mit reduzierter Adhäsionskraft 82 auf der Oberfläche anhaften. Im Vergleich zu einer unbehandelten Oberfläche kann es so zu einer geringeren bzw. verlangsamten Zellprofileration (Biofilmbildung 84) kommen. Dieser Effekt resultiert aus einer verringerten "biological contact width" 87 (siehe Fig. 4). Teilweise kann es auch vorkommen, dass die Nanostruktur die Bakterien 81 abtötet (Fig. 4 rechts), wobei ersteres als anti-adhäsiver Effekt und letzteres als bakterizider Effekt der Nanostruktur 40 bezeichnet wird. Um diesen gewünschten anti-adhäsiven Effekt zu erzeugen, müssen die Spitzen 50 der Nanopartikel mit geeigneten Spitzenabständen 46 ausgebildet sein. Ist dieser Spitzenabstand 46 zu groß, so kann kein anti-adhäsiver Effekt mehr erzielt werden. Ist der Spitzenabstand 46 hingegen zu klein, so wurde beobachtet, dass dann der bakterizide Effekt der Nanostruktur 40 abnimmt, was ebenfalls unerwünscht ist.

Die Erfindung sieht nun, nach Durchführung der Erzeugung der Nanostruktur 40, einen zusätzlichen Prozessschritt B vor, der die Haftung der bereits auf der Oberfläche 5 erzeugten und darauf haftenden Nanostruktur 40 wesentlich verbessert.

Unter einer abriebfesten Anbindung der Nanostruktur 40 an die Oberfläche 5 des Trägerkörpers 2 kann hier insbesondere eine nano-mechanische Verbindung verstanden werden, die gegenüber mechanischem Abrieb für die angedachte Nutzung ausreichend stabil ist. Ein solcher Abrieb wird gewöhnlich beispielsweise bei Implantaten 1 erzeugt, wenn das Implantat 1 mit seiner Oberfläche 5 in Kontakt mit Knochen gerät und dabei Scherkräfte zwischen dem Knochen und der Implantatoberfläche auftreten. Das erfindungsgemäße Verfahren kann bei geeigneter Wahl der Prozessparameter eine so hohe Abriebfestigkeit der Nanostruktur 40 zum Beispiel bei Verwendung auf Titanimplantaten 1 gewährleisten, dass sich der Knochen an der Nanostruktur 40 abreibt und nicht umgekehrt, ohne dass die Nanostruktur 40 dabei in großen Teilen oder gar vollständig zerstört wird. In einem weiteren Beispiel ist die Abriebfestigkeit der Nanostruktur 40 so hoch, dass unter Druck-/Scherbelastung nur einzelne Nanopartikel 40 aus dieser herausbrechen, aber die Nanostruktur 40 nicht im Gesamten von der Oberfläche 5 abgelöst wird. Damit wird sichergestellt, dass die von der Nanostruktur 40 gelieferten Oberflächeneigenschaften, insbesondere eine Bakterienabweisende und/oder bakterizide Wirkung, auch bei einem Einsatz z.B. in der Zahnmedizin oder in der Endoprothetik dauerhaft zum Tragen kommen.

In dem zweiten Prozessschritt B wird die Nanostruktur 40 auf der Oberfläche 5 dauerhaft und abriebfest "fixiert", in dem eine Einbettschicht 20 an der Oberfläche 5, und damit ein Schichtverbund 60, erzeugt wird. Dabei bedeckt die Einbettschicht 20 die Nanopartikel 40 nicht vollständig, das heißt die jeweilige Mantelfläche 51 der Nanopartikel 40 ist nicht vollständig von der Einbettschicht 20 umhüllt, sondern die Einbettschicht 20 wird hauptsächlich an der Grenzfläche zwischen Nanostruktur 40 und der Grenzfläche 14 des Trägerkörpers 2 zu seiner Oxidschicht 10 erzeugt.

Die Einbettschicht 20 kann beispielsweise durch thermische oder oxidative Schichtwachstumsverfahren, also insbesondere unter chemischer Umwandlung von Teilen der Oberfläche 14 des Trägerkörpers 2, oder mittels eines Depositionsverfahrens aufgebracht werden: Im ersten Fall kann eine Umwandlung der obersten Werkstoffschicht von Metall zu z.B. Metalloxid oder Metallnitrid erfolgen, was in der Regel mit einer Volumenänderung einhergeht. Daher kommt es zu einem einwachsenden Anteil 21, gemessen von der Ursprungsoberfläche 5, und einem aufwachsenden Anteil 24. Im zweiten Fall kann die gesamte Einbettschicht 20 aus einem Material bestehen, welches auf der Oberfläche 14 bzw. 5 des Trägerkörpers 2 abgeschieden wurde, beispielsweise mittels physikalischem Verdampfen (PVD), chemischer Dampfphasendeposition (CVD) oder mittels sonstiger Verfahren.

Die Einbettschicht 20 bettet also die Nanostrukturen 40 nicht vollständig ein, sondern nur teilweise. Denn erkennbar ragt ein Teil 49 der Nanostruktur 40 aus der Einbettschicht 20 heraus. Dieser Teil ist für die erhaltenen neuen Oberflächeneigenschaften ursächlich.

Insbesondere kann die Erzeugung der Einbettschicht 20 durch eine elektrochemische Anodisation erfolgen. Bei der Anodisation liegt die eingesetzte elektrische Spannung unterhalb einer Durchbruchspannung des Materials 3 des Trägerkörpers 2. Die elektrochemisch Anodisation der nanostrukturierten Oberfläche führt zu einer sehr homogenen Erzeugung der Einbettschicht 20, auch auf komplex geformten Trägerkörpern 2. Dadurch kann eine konforme Oberflächenbeschichtung realisiert werden. Die Verwendung niedriger Spannungen und kurzer Prozessdauern macht das Verfahren dabei besonders einfach in der Anwendung sowie kostengünstig, da das Verfahren mit simplen Apparaturen durchführbar ist und hohen Durchsatz erlaubt. Zudem ist dieses Verfahren schonend anwendbar für komplex geformte Trägerkörper 2. Grundsätzlich kann bei der elektrochemischen Oxidation auch mit einer elektrischen Wechselspannung unter Verwendung geeigneter Parameter ein vergleichbares Ergebnis erzielt werden. Bevorzugt ist aber eine elektrochemische Oxidation unter Verwendung einer Gleichspannung, weil dadurch eine einfachere Prozesskontrolle möglich ist.

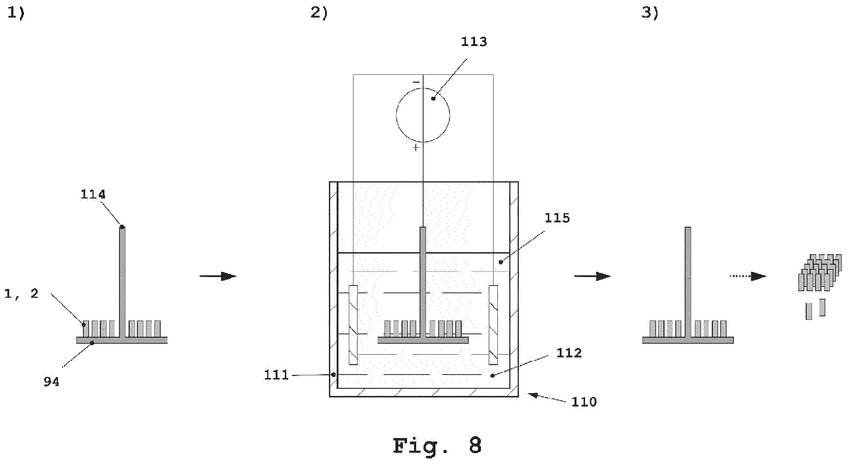

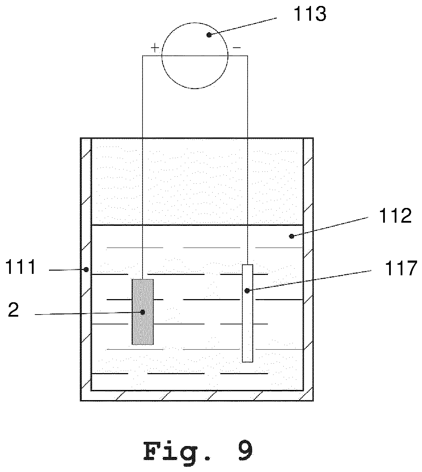

Der Prozessschritt B mittels elektrochemischer Anodisation ist schematisch in den Figuren 8 und 9 gezeigt. Bei der elektrochemischen Anodisation wird der Trägerkörper 2 mit der Nanostruktur 20 auf einen Halter 94 geladen (Schritt 1) in Fig. 8) und mit samt dem Halter 94 in ein Gefäß 99 befüllt mit einem flüssigen Elektrolyten 100, zum Beispiel einprozentige Schwefelsäure (H2SO4), eingetaucht. Der flüssige Elektrolyt 100 enthält keine Fluor-haltige Verbindung und ist somit Fluor-frei. Es wird dann mittels einer Spannungsquelle 113 eine Gleichspannung unterhalb von 20 V angelegt (Schritt 2) in Fig. 8), beispielsweise eine Spannung von 8 V für 90 Sekunden. Die Spannung soll dabei unterhalb der Durchbruchspannung des Grundmaterials 3 des Trägerkörpers 2 liegen. Die Badeigenschaften 115 können dabei variieren, insbesondere Temperatur, Bewegung und Konzentration des Elektrolyten.

Der Halter 94 kann so ausgestaltet sein, dass es sowohl in Prozessschritt A als auch in Prozessschritt B verwendet werden kann und er eine Möglichkeit 114 bietet, die Trägerkörper 2 elektrisch zu kontaktieren und mit der Spannungsquelle zu verbinden.

Figur 11 zeigt schematisch die Abhängigkeit der Dicke der Einbettschicht 20 von der Höhe der Gleichspannung im Schritt B. Wenn Spannungen oberhalb dieses Grenzwerts verwendet werden, beispielsweise von 20 V bei Verwendung eines Titan-Trägerkörpers, so wurde beobachtet, dass die gesamte Nanostruktur von der Einbettschicht überwachsen ist, sodass dann die nanoskopischen Spitzen 50 der Nanopartikel 40 praktisch vollständig oder sogar vollständig in der Einbettschicht 20 verschwinden (ganz rechts in Fig. 11) und entsprechend die gewünschten Effekte nicht länger erzielt werden.

Ein besonders günstiges Prozessfenster liegt bei einer DC-Spannung von 6-10 V, was beispielsweise oftmals ausreichend ist, um eine Schichtdicke 21 der Einbettschicht von 10-20 nm auszubilden (drittes von oben in Fig. 11). Die exakten Werte sind dabei auch von der Reaktionstemperatur und der Elektrolytkonzentration und der Ausgangsrauigkeit der Trägerkörper 2 abhängig. Wenn sehr geringe Spannungen verwendet werden, so wurde beobachtet, dass die Einbettschicht 20 nicht gebildet wird (oben in Fig. 11) oder, bei beispielsweise 5 V, eine zu geringe Schichtdicke aufweist, um einen Verankerungseffekt zu erzeugen (zweite Abbildung von oben in Fig. 11). Nach Ablauf des Prozesses wird der Trägerkörper aus der Reaktionsflüssigkeit entladen (Schritt 3) in Fig. 8) und vom Halter 94 entfernt (Schritt 4) in Fig. 8). Es können zwischen den hier dargestellten Prozessschritten selbstverständlich weitere Arbeitsschritte, insbesondere Reinigungsschritte, Trocknungs- oder Temperierungsschritte, durchgeführt werden.

Genauer ist es so, dass vor Anwendung des Prozessschrittes B die Nanopartikel 40 beispielsweise eine durchschnittliche Höhe 43 aufweisen können. Es ist dann bevorzugt, dass die Prozessführung im Oxidationsverfahren B so gewählt ist, dass wenigstens 20 %, bevorzugt wenigstens 50 % und besonders bevorzugt wenigstens 80 % der Höhe 43 der Nanopartikel 40 am Ende des Verfahrens noch aus der Einbettschicht 20 hervorstehen. Mit anderen Worten soll also die Dicke 21 der Einbettschicht 20, gemessen von der ursprünglichen Oberfläche 5 vor Durchführung des Oxidationsverfahrens B, einen Wert von 80 %, vorzugsweise 50 %, besonders bevorzugt sogar von nur 20 % der ursprünglichen Höhe 43 der Nanostruktur 40 (erneut gemessen oberhalb der ursprünglichen Oberfläche 5) erreichen.

Bei dem erläuterten Prozess erfolgt eine Umwandlung der obersten Werkstoffschicht von Titan zu Titanoxid. Bei diesem Prozess wächst die Oxidschicht 10 sowohl in das Grundmaterial 3 hinein (gemessen von der ursprünglichen Ausgangsoberfläche 5 vor dem Prozess) und bildet so einen eingewachsenen Anteil 22 der Einbettschicht 20 aus. Dies wird möglich durch die Volumensteigerung bei der Umwandlung aus dem Grundmaterial 3 heraus. Entsprechend bildet die Einbettschicht auch einen aufgewachsenen Anteil 24 der Einbettschicht 20 aus. Das Ein- und Aufwachsen der Titanschicht auf die Oberfläche 5 des Trägerkörpers 2 ist auch in Figur 10 (links) schematisch gezeigt. Insbesondere kann die eingewachsene Schichtdicke 23 ca. 16-24 nm betragen. Durch die Einbettschicht 20 wird damit die Abriebfestigkeit erzeugt.

Die maximale aufgewachsene Schichtdicke 25 ("Einbetthöhe"), in der Figur als D2 bezeichnet, kann dabei vorzugsweise kleiner sein als eine Gesamtdicke 21 der Einbettschicht 20, sofern die Einbettschicht 20 auch in den Trägerkörper 2 hineinwächst und sich somit nach innen über die ursprüngliche Oberfläche 4, bzw. 14, hinaus ausbreitet (also z.B. in einen als Trägerkörper 2 verwendeten Körper aus Titan hineinwächst). Dies hängt davon ab, mit welchem Verfahren die Einbettschicht 20 auf die Oberfläche 4/5 aufgebracht wird. Ferner ist es wie erwähnt bevorzugt, wenn diese Einbetthöhe D2 weniger als 50% einer maximalen Höhe 102 der Nanostruktur 40 aufweist, weil dann die gewünschten Oberflächeneffekte zum Tragen kommen.

Bei Verwendung eines Titankörpers als Trägerkörper 2, auf dem die Nanostruktur 40 im Schritt A aufgewachsen wurde, wird mit dem Oxidationsverfahren B eine amorphe Titanoxidschicht 24 aufgewachsen, sodass in der Einbettschicht 20 also gerade keine kristallinen Nanopartikel ausgebildet werden. Diese amorphe Titanschicht weist insbesondere Titandioxid auf.

Aufgrund ihrer kristallinen Struktur und der damit einhergehenden chemischen Inertheit wird die in Prozessschritt A hergestellte Nanostruktur 40 hingegen im Prozessschritt B nicht mehr in ihrer Morphologie, Form, Stöchiometrie und/oder Kristallographie verändert. Dies kann insbesondere dann gewährleistet sein, wenn die Nanostruktur 40 nach Herstellung durch Prozessschritt A aus TiO₂-Kristallen (z.B. Anatas) besteht, welche chemisch sehr inert sind und insbesondere stabil gegenüber Prozessschritt B sind. Die Einbettschicht 20 kann also insbesondere chemisch unedler (d.h. weniger chemisch stabil) als die Nanostruktur 40 sein, wobei sowohl Nanostruktur 40 als auch Einbettschicht 20 zusätzlich durch andere Materialien dotiert sein können.

Weiterhin wird die Nanostruktur 40 insbesondere dann nicht verändert, wenn in Prozessschritt B, wie oben beschrieben, keine Fluor-halte Verbindung, also insbesondere ein fluorfreier Elektrolyt, eingesetzt wird. Mit anderen Worten modifiziert der Prozessschritt B selektiv nur die ursprüngliche Oberfläche 5 des Trägerkörpers 2, weil die Einbettschicht aus dieser Oberfläche 5 bzw. aus dem Trägerkörper 2 heraus und ggf. in diese Oberfläche 5 bzw. in den Trägerkörper 2 hinein wächst, aber der Prozessschritt B modifiziert nicht die Nanostruktur 40.

Da sich aufgrund der Interferenzeffekte die Farbe des Implantats 1 nach Durchführung des Oxidationsverfahrens B ändern kann, sieht eine besonders bevorzugte Ausgestaltung vor, dass dann, wenn das Verfahren für Zahn-Implantate verwendet wird, die Spannung auf 8-10 V begrenzt wird. Denn so kann eine Verfärbung des Implantats 1, die im Zahnbereich störend wäre, vermieden werden.

Zusammenfassend wird ein Verfahren zum Erzeugen einer abriebfesten Nanostruktur 40 auf einer Oberfläche 5 eines Trägerkörpers 2 vorgeschlagen, um den Trägerkörper 2 so dauerhaft mit vorteilhaften Oberflächeneffekten auszustatten. Das Verfahren lässt sich dabei insbesondere einsetzen, um Nanostrukturen 40 dauerhaft auf einer Oberfläche 5 eines dauerhaften oder temporären Implantats abriebfest nanomechanisch zu verankern; es kann aber auch auf anderen Oberflächen 5 zum Einsatz kommen, etwa um Benetzungseigenschaften zu modifizieren. Hierzu wird in einem ersten Prozessschritt A die Nanostruktur 40 zunächst auf die Oberfläche 5 aufgebracht bzw. auf dieser erzeugt oder aufgewachsen. In einem nachfolgenden Prozessschritt B wird dann die in Schritt A erzeugte Nanostruktur 40 mittels einer separaten Einbettschicht 20 dauerhaft und abriebfest an die Oberfläche 5 angebunden, wobei die Einbettschicht 20 Zwischenräume zwischen den erzeugten Nanostrukturen 40 auffüllt und somit auf die Nanostruktur 40 aufwächst. Durch ein solches Aufwachsen (aus dem Trägerkörper 2 heraus) kann insbesondere eine komplementäre Form der Einbettschicht 20 entstehen. Hierzu wird die Einbettschicht 20 separat von der bereits erzeugten Nanostruktur 40 an, insbesondere auf und/oder in, der Oberfläche 5 erzeugt, wobei die Nanostruktur 40 im Prozessschritt B nicht modifiziert wird. Die Einbettschicht 20 bedeckt die Nanostruktur 40 dabei aber nur teilweise, sodass Oberflächeneigenschaften der Nanostruktur 40 zumindest teilweise erhalten werden können. Gleichzeitig verankert die Einbettschicht 20 die Nanostruktur 40 auf der Oberfläche 5, wobei die Einbettschicht 20 hierzu, insbesondere durch die Nanostruktur 20 hindurch, in den Trägerkörper 2 einwachsen kann.

### Bezugszeichenliste

- 1: Implantat, Titankörper
- 2: Trägerkörper, Grundkörper
- 3: Grundmaterial
- 4: Gefüge des Grundmaterials
- 5: Ausgangsoberfläche
- 6: Oberflächenrauigkeit
- 7: Resultierende neue Oberfläche
- 10: Ggf. vorhandene Oxidschicht
- 11: Material der ggf. vorhandenen Oxidschicht
- 12: Gefüge der ggf. vorhandenen Oxidschicht
- 13: Dicke der ggf. vorhandenen Oxidschicht
- 14: Grenzfläche zwischen ggf. vorhandener Oxidschicht und Grundmaterial des Grundkörpers
- 20: Einbettschicht
- 21: Gesamt-Dicke der Einbettschicht
- 22: eingewachsener Anteil der Einbettschicht
- 23: Dicke des eingewachsenen Anteils der Einbettschicht
- 24: aufgewachsener Anteil der Einbettschicht
- 25: Dicke des aufgewachsenen Anteils der Einbettschicht
- 26: Grenzfläche zwischen ggf. vorhandener Oxidschicht und eingewachsener Einbettschicht
- 27: Grenzfläche zwischen ggf. vorhandener Oxidschicht und aufgewachsener Einbettschicht
- 28: Wachstumsrichtung der einwachsenden Einbettschicht
- 29: Wachstumsrichtung der aufwachsenden Einbettschicht
- 30: Einbettbereich der Nanostrukturen durch die Einbettschicht
- 31: Bereich der Einbettschicht zwischen den Nanostrukturen
- 32: Oberfläche der Einbettschicht
- 33: Material der Einbettschicht
- 34: Gefüge der Einbettschicht
- 35: resultierender Verbindungsbereich zwischen Einbettschicht 20 und Nanostruktur 40
- 40: Nanostrukturen (oder Nanopartikel)
- 41: Material der Nanostrukturen
- 42: Gefüge der Nanostrukturen
- 43: Durchschnittliche Höhe der Nanostrukturen
- 44: Durchschnittliche Breite der Nanostrukturen
- 45: Durchschnittliche Spitzengröße bzw. Spitzendurchmesser
- 46: Durchschnittlicher Abstand zweier Nanostrukturen
- 47: Mittellinie, Normalenrichtung der Nanostrukturen
- 48: Öffnungswinkel der Nanostrukturen
- 49: Aus der Einbettschicht herausragender Teil der Nanostrukturen
- 50: Spitze der Nanostrukturen
- 51: Oberfläche, Mantelfläche der Nanostrukturen
- 52: Kontaktfläche zwischen Nanostruktur 40 und ursprünglicher Oberfläche 5
- 60: Schichtverbund
- 70: Verfahren zur Herstellung einer abriebfesten Nanostruktur
- 71-73: Schritte des Verfahrens 70
- 81: Bakterium
- 82: Adhäsionskräfte
- 83: Antiadhäsive Eigenschaften
- 84: Biofilmbildung
- 85: Bakterizide Effekte
- 86: Interaktion Bakterium/Oberfläche
- 87: Interaktionsbereich, Tragfläche
- 90: Druckreaktor
- 91: Deckel
- 92: Verschluss
- 93: Grundgefäß
- 94: PTFE-Gestell
- 95: PTFE-Inlet
- 96: Wässrige Lösung
- 97: Füllstand
- 98: Gasgemisch
- 99: Druck
- 100: Heizsystem/Mantelheizung/Ofen
- 101: Abstoßungswirkung (repelling effect)
- 102: maximale Höhe (von 40 gemessen über 5)
- 103: Einbetthöhe D2
- 110: Vorrichtung für Prozessschritt B
- 111: Prozessbecken, Gefäß
- 112: Elektrolyt
- 113: Spannungsquelle
- 114: Adapter für elektrische Kontaktieren
- 115: Badeigenschaften
- 116: Zwischenarbeitsschritte
- 117: Gegenelektrode
- 120: Gefügeeigenschaften, Gefügeaufbau des Grundmaterials 1
- 121: Körner des Grundmaterials
- 122: Korngrenzen
- 123: Normalen zur Kristallorientierung

## Patentansprüche

1. **Verfahren zum Erzeugen einer abriebfesten Nanostruktur (40)** auf einer Oberfläche (5), insbesondere auf Teilbereichen der Oberfläche (5), eines Trägerkörpers (2), insbesondere in Form eines dauerhaften oder temporären Implantats (1),
- wobei in einem ersten Prozessschritt A die Nanostruktur (40) auf die Oberfläche (5) aufgebracht, insbesondere auf der Oberfläche (5) aufgewachsen, wird,
**dadurch gekennzeichnet,**
- **dass** die im Prozessschritt A erzeugte Nanostruktur (40) in einem zweiten Prozessschritt B mittels einer Einbettschicht (20) dauerhaft und abriebfest an die Oberfläche (5) angebunden wird,
- wobei die Einbettschicht (20) an der Oberfläche (5) erzeugt, insbesondere auf der Nanostruktur (40) abgeschieden, wird
- wobei die Einbettschicht (20) die Nanostruktur (40) nur teilweise einbettet, und
- wobei die Einbettschicht (20) durch chemische Umwandlung einer obersten Werkstoffschicht des Trägerkörpers (2) unter Volumenzunahme erzeugt wird.

2. Verfahren nach Anspruch 1,
wobei eine innere Schichtdicke D1 der Einbettschicht (20), gemessen unterhalb der ursprünglichen Oberfläche (5) des Trägerkörpers (2), mindestens 6 nm, bevorzugt mindestens 12 nm, besonders bevorzugt mindestens 18 nm beträgt und/oder
- wobei die jeweilige Nanostruktur (40) konisch zulaufend ausgestaltet ist, insbesondere sodass eine Abstoßungswirkung (83,101) auf Mikroorganismen erzielt wird.

3. Verfahren nach einem der vorangehenden Ansprüche, wobei eine maximale Schichtdicke/Einbetthöhe D2 (25,103) der Einbettschicht (5), gemessen oberhalb der ursprünglichen Oberfläche (5) des Trägerkörpers (2), kleiner ist, vorzugsweise halb so hoch ist, als eine durchschnittliche Höhe H (43) der Nanostruktur (40), erneut gemessen oberhalb der ursprünglichen Oberfläche (5) des Trägerkörpers (2),
- insbesondere sodass die Nanostruktur (40) aus der Einbettschicht (20) herausragt,
- vorzugsweise wobei die Nanostruktur (40) mit wenigstens 20%, bevorzugt mit wenigstens 50%, besonders bevorzugt mit wenigstens 66% der durchschnittlichen Höhe H (43) aus der Einbettschicht (20) herausragt.

4. Verfahren nach einem der vorhergehenden Ansprüche, wobei ein aufgewachsener Anteil (24) der Einbettschicht (20) eine Form aufweist, die komplementär zu einer Form der Nanostruktur (40) ist,
- vorzugweise wobei die Einbettschicht (20) Zwischenräume zwischen einzelnen Nanostrukturen (40) auf der Oberfläche (5) teilweise ausfüllt und/oder
- wobei der aufgewachsene Anteil (24) der Einbettschicht (20) Aussparungen aufweist, die in ihrer Form einer jeweiligen Grundfläche der jeweiligen Nanostruktur (40) entsprechen und/oder die in Bezug auf eine Richtung einer Normalen der Oberfläche (5) komplementär zu einer konischen Gestalt der Nanostruktur (40) ausgestaltet sind.

5. Verfahren nach einem der vorhergehenden Ansprüche, wobei die Einbettschicht (20), vorzugsweise mittels eines elektrochemischen Verfahrens und/oder mittels eines Oxidationsverfahrens, so erzeugt wird, dass die Einbettschicht (20) sowohl in den Trägerkörper (2) hineinwächst als auch aus der ursprünglichen Oberfläche (5) herauswächst,
- insbesondere derart, dass nach Abschluss des Prozessschritts B die Einbettschicht (20) tiefer in den Trägerkörper (2) hinein reicht als die Nanostruktur (40), vorzugsweise sodass die Einbettschicht (20) die Nanostruktur (40) nicht nur einbettet, sondern auch im Trägerkörper (2) nanoskopisch mechanisch verankert.

6. Verfahren nach einem der vorhergehenden Ansprüche, wobei der erste Prozessschritt A eine hydrothermische Oxidation ist, die in einem Druckreaktor durchgeführt wird,
- insbesondere wobei während des Prozessschritts A die Nanostruktur (40) nur auf der Oberfläche (5), insbesondere auf einer nativen Oxidschicht des Trägerkörpers (2), aufwächst, nicht aber in den Trägerkörper (2) hinein,
- vorzugsweise unter Verwendung von Reinstwasser, oder von wässrigem Ammoniak oder von KOH als reaktiver Flüssigkeit,
- besonders bevorzugt wobei die Oberfläche (5) des Trägerkörpers (2), auf welcher die Nanostruktur (40) aufgebracht werden soll, während des Prozessschritts A vollständig in die reaktive Flüssigkeit eingetaucht bleibt,
- insbesondere wobei der Trägerkörper (2) aus Metall, insbesondere aus Titan, gefertigt ist, sodass durch die hydrothermische Oxidation ein Metalloxid, insbesondere Titanoxid, in Form von Nanopartikeln entsteht, welches/die die Nanostruktur (40) ausbildet/ausbilden.

7. Verfahren nach einem der vorhergehenden Ansprüche, wobei der zweite Prozessschritt B eine elektrochemische Anodisation zur Erzeugung der Einbettschicht (20) ist,
- vorzugsweise unter Verwendung eines flüssigen Elektrolyten wie beispielsweise verdünnte Schwefelsäure oder vergleichbaren geeigneten Elektrolyten und unter Einsatz einer elektrischen Gleichspannung, vorzugsweise von unter 20 V, bevorzugt von unter 15 V, besonders bevorzugt von unter 10 V und/oder
- wobei die elektrochemische Anodisation mit einer Einwirkzeit von unter 30 min, bevorzugt von unter 5 min, besonders bevorzugt von unter 2 min, ausgeführt wird.

8. Verfahren nach einem der Ansprüche 1 bis 6, wobei der zweite Prozessschritt B eine nasschemische Oxidation zur Erzeugung der Einbettschicht (20) ist, vorzugsweise unter Verwendung einer oxidierenden Flüssigkeit,
- besonders bevorzugt wobei die Oberfläche (5) des Trägerkörpers (2), auf welcher die Einbettschicht (20) in abgeschieden werden soll, während des Prozessschritts B vollständig mit samt der Nanostruktur (40) in die oxidierende Flüssigkeit eingetaucht bleibt.

9. Verfahren nach einem der Ansprüche 1 bis 6, wobei der zweite Prozessschritt B ein plasmagestütztes oder plasmainduziertes Verfahren zur Erzeugung der Einbettschicht (20) ist,
- vorzugsweise unter Verwendung eines Plasmas, welches Sauerstoff enthält.

10. Verfahren nach einem der vorhergehenden Ansprüche, wobei die mit dem Verfahren hergestellte Nanostruktur (40) Nanopartikel, insbesondere in Form von Nanokristallen, aufweist, deren Spitzen im Mittel gleichgerichtet, insbesondere normal, zu der Oberfläche (5) ausgerichtet sind,
- vorzugweise wobei die Nanopartikel eine überwiegend tetragonal pyramidale, tetragonal bi-pyramidale und/oder eine konische oder doppelkonische Grundform aufweisen und/oder
- wobei die Nanopartikel eine durchschnittliche Höhe H (43), gemessen oberhalb der ursprünglichen Oberfläche (5), von weniger als 300 nm, bevorzugt von 250 nm, besonders bevorzugt weniger als 175 nm aufweisen und/oder
- wobei die Nanopartikel einen durchschnittlichen Spitzenabstand von weniger als 300 nm, bevorzugt von weniger als 250 nm, besonders bevorzugt weniger als 150 nm, weiter besonders bevorzugt weniger als 75 nm aufweisen.

11. Verfahren nach einem der vorhergehenden Ansprüche, wobei der Trägerkörper (2) aus Metall, vorzugsweise aus Titan oder aus einer Titanlegierung, gefertigt ist und/oder
- wobei die in Prozessschritt B hergestellte Einbettschicht (20) eine amorphe, teilamorphe oder rein kristalline Titanoxidschicht ist und/oder
- wobei die in Prozessschritt A hergestellte Nanostruktur (40) durch eine, zumindest teilweise, kristalline Titanoxidschicht gebildet ist und/oder
- wobei die Oberfläche (5) des Trägerkörpers (2) eine Oxidschicht, vorzugsweise eine Titanoxidschicht (TiO2 oder TiOx), aufweist, und die Nanostruktur (40) auf diese Oxidschicht aufgebracht oder aus dieser durch Umwandlung hervorgebracht wird.

12. **Schichtverbund (60) umfassend eine Nanostruktur (40),** wobei die Nanostruktur (40) abriebfest auf einer Oberfläche (5) eines Trägerkörpers (2) aufgebracht ist,
- vorzugsweise wobei die Nanostruktur (40) mittels eines Verfahrens nach einem der vorhergehenden Ansprüche erzeugt, insbesondere auf dem Trägerkörper (2) aufgebracht oder aufgewachsen, wurde, **dadurch gekennzeichnet,**
- **dass** die Nanostruktur (40) Nanopartikel, insbesondere in Form von Nanokristallen, aufweist, die wenigstens einen oberflächenaktiven Effekt, beispielsweise
- einen optischen und/oder
- einen anti-adhäsiven Effekt und/oder
- einen bakteriziden Effekt,
erzeugen,
- **dass** die Nanostruktur (40) zumindest teilweise in eine Einbettschicht (20) eingebettet ist, wobei die Nanostruktur (40) aus der Einbettschicht (20) so weit hervorsteht, dass wenigstens ein oberflächenaktiver Effekt zumindest teilweise erhalten bleibt, und
- **dass** die Einbettschicht (20) einen aufgewachsenen Anteil (24) oberhalb der Oberfläche (5) sowie einen eingewachsenen Anteil (22) unterhalb der Oberfläche (5) aufweist.

13. Schichtverbund (60) gemäß dem vorhergehenden Anspruch, wobei die Einbettschicht (20) über die Nanostruktur (40) nach innen in Richtung des Trägerkörpers (2) hinaus reicht,
- insbesondere sodass die Einbettschicht (20) tiefer in den Trägerkörper (2) hineinreicht im Vergleich zur Nanostruktur (40),
- vorzugsweise sodass eine nanomechanische Verankerung der Nanostruktur (40) auf einer Oberfläche (5) des Trägerkörpers (2) und/oder in dem Trägerkörper (2) erzielt ist und/oder
- wobei sich die Einbettschicht (20) mindestens morphologisch von der Nanostruktur (40) unterscheidet, insbesondere wobei die Einbettschicht (20) und die Nanostruktur (40) aus demselben Material, insbesondere aus einem Metalloxid, hergestellt sind.

14. **Trägerkörper (2),** insbesondere in Form eines Implantats (1), **mit einem Schichtverbund (60) gemäß einem der vorhergehenden Ansprüche 12 oder 13,**
- vorzugsweise wobei sich die Einbettschicht (20) morphologisch von der Nanostruktur (40) unterscheidet und/oder
- wobei die Einbettschicht (20) zumindest teilweise in den Trägerkörper (2) eingewachsen wurde/ist.

15. **Verwendung eines Schichtverbunds (60) nach einem der Ansprüche 12 oder 13** auf einem Trägerkörper (2) zur Erzeugung maßgeschneiderter Oberflächeneigenschaften des Trägerkörpers (2),
- vorzugsweise wobei der Schichtverbund (60) mit einem Verfahren nach einem der Ansprüche 1-11 hergestellt wurde und/oder mit einem solchen Verfahren auf einer Oberfläche (5) des Trägerkörpers (2) abriebfest verankert wurde,
- insbesondere sodass bei ausreichendem Druck ein zugehöriger Kontaktkörper des Trägerkörpers (2), beispielsweise ein Knochen, an der Nanostruktur (40) abgerieben wird und nicht die Nanostruktur (40) an dem Kontaktkörper, wenn der Trägerkörper (2) unter dem Druck mit samt dem Schichtverbund (60) auf dem Kontaktkörper entlang gleitet,
- vorzugsweise wobei der Trägerkörper (2) ein Implantat (1) ist und wobei der Schichtverbund (60) eine bakterizide Wirkung und/oder einen anti-adhäsiven Effekt in Bezug auf die äußerste Oberfläche des Implantats (1), die durch die Nanostruktur (40) mit ausgebildet wird, erzeugt.

## Claims

1. **Method for producing an abrasion-resistant nanostructure (40)** on a surface (5), in particular on portions of the surface (5), of a carrier body (2), in particular in the form of a permanent or temporary implant (1),
- wherein, in a first process step A, the nanostructure (40) is applied to the surface (5), in particular grown on the surface (5),
**characterized in that**
- the nanostructure (40) produced in process step A is bonded permanently and in an abrasion-resistant manner to the surface (5) in a second process step B by means of an embedding layer (20),
- wherein the embedding layer (20) is produced on the surface (5), in particular deposited on the nanostructure (40)
- wherein the embedding layer (20) only partially embeds the nanostructure (40), and
- wherein the embedding layer (20) is produced by chemical conversion of an uppermost material layer of the carrier body (2) with an increase in volume.

2. Method according to claim 1,
wherein an inner layer thickness D1 of the embedding layer (20), measured below the original surface (5) of the carrier body (2), is at least 6 nm, preferably at least 12 nm, particularly preferably at least 18 nm, and/or
- wherein the respective nanostructure (40) is designed to taper conically, in particular so that a repellent effect (83, 101) on microorganisms is achieved.

3. Method according to one of the preceding claims, wherein a maximum layer thickness/embedding height D2 (25, 103) of the embedding layer (5), measured above the original surface (5) of the carrier body (2), is smaller, preferably half as high as an average height H (43) of the nanostructure (40), measured again above the original surface (5) of the carrier body (2),
- in particular so that the nanostructure (40) protrudes from the embedding layer (20),
- preferably wherein the nanostructure (40) protrudes from the embedding layer (20) by at least 20%, preferably by at least 50%, particularly preferably by at least 66% of the average height H (43).

4. Method according to one of the preceding claims, wherein a grown portion (24) of the embedding layer (20) has a shape that is complementary to a shape of the nanostructure (40),
- preferably wherein the embedding layer (20) partially fills intermediate spaces between individual nanostructures (40) on the surface (5) and/or
- wherein the grown portion (24) of the embedding layer (20) has recesses which correspond in shape to a respective base area of the respective nanostructure (40) and/or which are designed to be complementary to a conical shape of the nanostructure (40) with respect to a direction of a normal to the surface (5).

5. Method according to one of the preceding claims, wherein the embedding layer (20) is produced, preferably by means of an electrochemical process and/or by means of an oxidation process, in such a way that the embedding layer (20) grows both into the carrier body (2) and out of the original surface (5),
- in particular such that, after completion of process step B, the embedding layer (20) extends deeper into the carrier body (2) than the nanostructure (40), preferably such that the embedding layer (20) not only embeds the nanostructure (40) but also anchors it mechanically in the carrier body (2) at the nanoscale.

6. Method according to one of the preceding claims, wherein the first process step A is a hydrothermal oxidation carried out in a pressure reactor,
- in particular wherein during process step A the nanostructure (40) only grows on the surface (5), in particular on a native oxide layer of the carrier body (2), but not into the carrier body (2),
- preferably using ultrapure water, or aqueous ammonia or KOH as the reactive liquid,
- particularly preferably wherein the surface (5) of the carrier body (2) onto which the nanostructure (40) is to be applied remains completely immersed in the reactive liquid during process step A,
- in particular wherein the carrier body (2) is made of metal, in particular titanium, so that the hydrothermal oxidation produces a metal oxide, in particular titanium oxide, in the form of nanoparticles, which forms/form the nanostructure (40).

7. Method according to one of the preceding claims, wherein the second process step B is an electrochemical anodization to produce the embedding layer (20),
- preferably using a liquid electrolyte such as diluted sulfuric acid or comparable suitable electrolytes and applying a direct electric voltage, preferably of less than 20 V, preferably of less than 15 V, particularly preferably of less than 10 V, and/or
- wherein the electrochemical anodization is carried out with an exposure time of less than 30 minutes, preferably less than 5 minutes, particularly preferably less than 2 minutes.

8. Method according to one of claims 1 to 6, wherein the second process step B is a wet chemical oxidation to produce the embedding layer (20), preferably using an oxidizing liquid,
- particularly preferably wherein the surface (5) of the carrier body (2) on which the embedding layer (20) is to be deposited remains completely immersed in the oxidizing liquid together with the nanostructure (40) during process step B.

9. Method according to one of claims 1 to 6, wherein the second process step B is a plasma-assisted or plasma-induced method for producing the embedding layer (20),
- preferably using a plasma containing oxygen.

10. Method according to one of the preceding claims, wherein the nanostructure (40) produced by the method has nanoparticles, in particular in the form of nanocrystals, whose tips are on average aligned in the same direction, in particular normal, to the surface (5),
- preferably wherein the nanoparticles have a predominantly tetragonal pyramidal, tetragonal bi-pyramidal and/or conical or double conical basic shape and/or
- wherein the nanoparticles have an average height H (43), measured above the original surface (5), of less than 300 nm, preferably 250 nm, particularly preferably less than 175 nm, and/or
- wherein the nanoparticles have an average tip-to-tip distance of less than 300 nm, preferably less than 250 nm, particularly preferably less than 150 nm, and further particularly preferably less than 75 nm.

11. Method according to one of the preceding claims, wherein the carrier body (2) is made of metal, preferably of titanium or of a titanium alloy, and/or
- wherein the embedding layer (20) produced in process step B is an amorphous, partially amorphous or purely crystalline titanium oxide layer, and/or
- wherein the nanostructure (40) produced in process step A is formed by an at least partially crystalline titanium oxide layer and/or
- wherein the surface (5) of the carrier body (2) has an oxide layer, preferably a titanium oxide layer (TiO₂ or TiOₓ), and the nanostructure (40) is applied to this oxide layer or produced from it by conversion.

12. **Layer composite (60) comprising a nanostructure (40),** wherein the nanostructure (40) is applied in an abrasion-resistant manner to a surface (5) of a carrier body (2),
- preferably wherein the nanostructure (40) has been produced by means of a method according to one of the preceding claims, in particular applied or grown on the carrier body (2),
**characterized**
- **in that** the nanostructure (40) comprises nanoparticles, in particular in the form of nanocrystals, which generate at least one surface-active effect, for example
- an optical and/or
- an anti-adhesive effect and/or
- a bactericidal effect,
- **in that** the nanostructure (40) is at least partially embedded in an embedding layer (20), wherein the nanostructure (40) protrudes from the embedding layer (20) to such an extent that at least one surface-active effect is at least partially retained, and
- **in that** the embedding layer (20) has a grown-on portion (24) above the surface (5) and a grown-in portion (22) below the surface (5).

13. Layer composite (60) according to the preceding claim, wherein the embedding layer (20) extends inwardly toward the carrier body (2) beyond the nanostructure (40),
- in particular so that the embedding layer (20) extends deeper into the carrier body (2) than the nanostructure (40),
- preferably so that nanomechanical anchoring of the nanostructure (40) on a surface (5) of the carrier body (2) and/or in the carrier body (2) is achieved and/or
- wherein the embedding layer (20) differs at least morphologically from the nanostructure (40), in particular wherein the embedding layer (20) and the nanostructure (40) are made of the same material, in particular of a metal oxide.

14. **Carrier body (2),** in particular in the form of an implant (1), **having a layer composite (60) according to one of the preceding claims 12 or 13,**
- preferably wherein the embedding layer (20) differs morphologically from the nanostructure (40) and/or
- wherein the embedding layer (20) has been/is at least partially grown into the carrier body (2).

15. **Use of a layer composite (60) according to one of claims 12 or 13** on a carrier body (2) for producing customized surface properties of the carrier body (2),
- preferably wherein the layer composite (60) has been produced by a method according to one of claims 1-11 and/or has been anchored in an abrasion-resistant manner on a surface (5) of the carrier body (2) by such a method,
- in particular so that, under sufficient pressure, an associated contact body of the carrier body (2), for example a bone, is abraded on the nanostructure (40) and not the nanostructure (40) on the contact body when the carrier body (2) slides along the contact body under the pressure together with the layer composite (60),
- preferably wherein the carrier body (2) is an implant (1) and wherein the layer composite (60) produces a bactericidal effect and/or an anti-adhesive effect with respect to the outermost surface of the implant (1), which is also formed by the nanostructure (40).

## Revendications

1. **Procédé de production d'une nanostructure (40) résistante** à **l'abrasion** sur une surface (5), en particulier sur des zones partielles de la surface (5), d'un corps de support (2), en particulier sous forme d'implant (1) permanent ou temporaire,
- la nanostructure (40) étant, dans une première étape A du procédé, appliquée sur la surface (5), en particulier développée sur la surface (5),
**caractérisé en ce que**
- la nanostructure (40) produite dans l'étape A du procédé est attachée, dans une deuxième étape B du procédé, de manière permanente et résistante à l'abrasion, à la surface (5) au moyen d'une couche d'enrobage (20),
- la couche d'enrobage (20) étant produite sur la surface (5), en particulier déposée par séparation sur la nanostructure (40),
- la couche d'enrobage (20) n'enrobant que partiellement la nanostructure (40), et
- la couche d'enrobage (20) étant produite par transformation chimique d'une couche de matériau supérieure du corps de support (2) en présence d'une augmentation de volume.

2. Procédé selon la revendication 1,
une épaisseur de couche intérieure D1 de la couche d'enrobage (20), mesurée au-dessous de la surface (5) initiale du corps de support (2), étant d'au moins 6 nm, de préférence d'au moins 12 nm, de façon particulièrement préférée d'au moins 18 nm, et/ou
- la nanostructure (40) respective étant constituée en se terminant de façon conique, en particulier de telle sorte qu'un effet de répulsion (83, 101) sur des microorganismes est obtenu.

3. Procédé selon l'une des revendications précédentes, une épaisseur de couche/hauteur d'enrobage D2 (25, 103) maximale de la surface (5), mesurée au-dessus de la surface (5) initiale du corps de support (2) étant plus petite, de préférence deux fois plus petite, qu'une hauteur H (43) moyenne de la nanostructure (40), mesurée ici aussi au-dessus de la surface (5) initiale du corps de support (2),
- en particulier de telle sorte que la nanostructure (40) dépasse de la couche d'enrobage (20),
- de préférence, la nanostructure (40) dépassant de la couche d'enrobage (20) d'au moins 20 %, de préférence d'au moins 50 %, de façon particulièrement préférée d'au moins 66 % de la hauteur H (43) moyenne.

4. Procédé selon l'une des revendications précédentes, une portion (24) développée de la couche d'enrobage (20) présentant une forme qui est complémentaire d'une forme de la nanostructure (40),
- de préférence la couche d'enrobage (20) comblant partiellement des interstices entre différentes nanostructures (40) sur la surface (5), et/ou
- la portion (24) développée de la couche d'enrobage (20) présentant des évidements qui, par leur forme, correspondent à une surface de base respective de la nanostructure (40) respective, et/ou qui sont constitués de façon complémentaire à une conformation conique de la nanostructure (40) par rapport à une direction d'une normale à la surface (5).

5. Procédé selon l'une des revendications précédentes, la couche d'enrobage (20) étant, de préférence au moyen d'un procédé électrochimique et/ou au moyen d'un procédé d'oxydation, produite de sorte que la couche d'enrobage (20) se développe aussi bien dans l'intérieur du corps de support (2) qu'à partir de la surface (5) initiale,
- en particulier de telle manière que, après achèvement de l'étape B de procédé, la couche d'enrobage (20) rentre plus profondément dans le corps de support (2) que la nanostructure (40), de préférence de telle sorte que la couche d'enrobage (20) non seulement enrobe la nanostructure (40) mais également la fixe mécaniquement de façon nanoscopique dans le corps de support (2).

6. Procédé selon l'une des revendications précédentes, la première étape A du procédé étant une oxydation hydrothermique qui est effectuée dans un réacteur sous pression,
- en particulier la nanostructure (40) se développant, pendant l'étape A du procédé, uniquement sur la surface (5), en particulier sur une couche d'oxyde native du corps de support (2) mais pas dans l'intérieur du corps de support (2),
- de préférence avec utilisation d'eau ultrapure, ou d'ammoniaque aqueuse ou de KOH comme liquide réactif,
- de façon particulièrement préférée la surface (5) du corps de support (2) sur laquelle la nanostructure (40) doit être appliquée demeurant complètement plongée dans le liquide réactif pendant l'étape A du procédé,
- en particulier le corps de support (2) étant réalisé en métal, en particulier en titane, de telle sorte que, du fait de l'oxydation hydrothermique, il est formé un oxyde métallique, en particulier de l'oxyde de titane, sous forme de nanoparticules qui constitue/constituent la nanostructure (40).

7. Procédé selon l'une des revendications précédentes, la deuxième étape B du procédé étant une anodisation électrochimique destinée à la production de la couche d'enrobage (20),
- de préférence avec utilisation d'un électrolyte comme par exemple de l'acide sulfurique dilué ou d'électrolytes appropriés comparables et en recourant à une tension continue électrique, de préférence inférieure à 20 V, préférablement inférieure à 15 V, de façon particulièrement préférée inférieure à 10 V, et/ou
- l'anodisation électrochimique étant réalisée avec un temps d'action inférieur à 30 min, préférablement inférieur à 5 min, de façon particulièrement préférée inférieur à 2 min.

8. Procédé selon l'une des revendications 1 à 6, la deuxième étape B du procédé étant une oxydation par voie humide destinée à la production de la couche d'enrobage (20), de préférence avec utilisation d'un liquide oxydant,
- de façon particulièrement préférée la surface (5) du corps de support (2) sur laquelle la couche d'enrobage (20) doit être déposée par séparation demeurant totalement plongée pendant la deuxième étape B du procédé dans le liquide oxydant avec la nanostructure (40).

9. Procédé selon l'une des revendications 1 à 6, la deuxième étape B du procédé étant un procédé, assisté par plasma ou induit par plasma, de production de la couche d'enrobage (20),
- de préférence avec utilisation d'un plasma qui contient de l'oxygène.

10. Procédé selon l'une des revendications précédentes, la nanostructure (40) fabriquée avec le procédé présentant des nanoparticules, en particulier sous forme de nanocristaux, dont les pointes sont en moyenne orientées dans le même sens, en particulier de façon normale à la surface (5),
- de préférence les nanoparticules présentant en particulier une forme de base principalement pyramidale tétragonale, bipyramidale tétragonale et/ou une forme de base conique ou biconique, et/ou
- de préférence les nanoparticules présentant une hauteur H (43) moyenne, mesurée au-dessus de la surface (5) initiale, de moins de 300 nm, de 250 nm, de façon particulièrement préférée de moins de 175 nm, et/ou
- les nanoparticules présentant un écart moyen entre pointes de moins de 300 nm, de préférence de moins de 250 nm, de façon particulièrement préférée de moins de 150 nm, de façon plus particulièrement préférée de 75 nm.

11. Procédé selon l'une des revendications précédentes, le corps de support (2) étant réalisé en métal, de préférence en titane ou en alliage de titane, et/ou
- la couche d'enrobage (20) fabriquée dans l'étape B de procédé étant une couche d'oxyde de titane amorphe, partiellement amorphe ou purement cristalline, et/ou
- la nanostructure (40) fabriquée dans l'étape A du procédé étant formée par une couche d'oxyde de titane au moins partiellement cristalline, et/ou
- la surface (5) du corps de support (2) présentant une couche d'oxyde, de préférence une couche d'oxyde de titane (TiO2 ou TiOx), et la nanostructure (40) étant appliquée sur cette couche d'oxyde ou étant produite à partir de celle-ci par transformation.

12. **Composé en couches (60) comprenant une nanostructure (40),** la nanostructure (40) étant appliquée de façon résistante à l'abrasion sur une surface (5) d'un corps de support (2),
- la nanostructure (40) ayant été de préférence produite au moyen d'un procédé selon l'une des revendications précédentes, en particulier appliquée ou développée sur le corps de support (2), **caractérisé en ce que**
- la nanostructure (40) présente des nanoparticules, en particulier sous forme de nanocristaux qui produisent au moins un effet tensioactif, par exemple
- un effet optique et/ou
- un effet antiadhésif et/ou
- un effet bactéricide,
- la nanostructure (40) est enrobée au moins partiellement dans une couche d'enrobage (20), la nanostructure (40) dépassant de la couche d'enrobage (20) suffisamment loin pour qu'au moins un effet tensioactif demeure conservé au moins partiellement, et
la couche d'enrobage (20) présente une portion (24) développée au-dessus de la surface (5) ainsi qu'une portion (22) développée au-dessous de la surface (5).

13. Composé en couches (60) selon la revendication précédente, la couche d'enrobage (20) s'étendant au-delà de la nanostructure (40) vers l'intérieur en direction du corps de support (2),
- en particulier de telle sorte que la couche d'enrobage (20) pénètre plus profondément dans le corps de support (2) en comparaison avec la nanostructure (40),
de préférence de telle sorte qu'une fixation nanomécanique de la nanostructure (40) sur une surface (5) du corps de support (2) et/ou dans le corps de support (2) est obtenue, et/ou
- la couche d'enrobage (20) se distinguant au moins morphologiquement de la nanostructure (40), la couche d'enrobage (20) et la nanostructure (40) étant en particulier fabriquées dans le même matériau, en particulier en oxyde métallique.

14. **Corps de support (2),** en particulier sous forme d'implant (1), **avec un composé en couches (60) selon l'une des revendications précédentes 12 ou 13,**
- de préférence la couche d'enrobage (20) se distinguant au moins morphologiquement de la nanostructure (40), et/ou
- la couche d'enrobage (20) ayant été/étant développée au moins partiellement dans le corps de support (2).

15. **Utilisation d'un composé en couches (60) selon l'une des revendications 12 ou 13** sur un corps de support (2) pour la production de caractéristiques superficielles conçues sur mesure du corps de support (2),
- de préférence le composé en couches (60) ayant été fabriqué selon l'une des revendications 1-11, et/ou ayant été fixé de façon résistante à l'abrasion avec un tel procédé sur une surface (5) du corps de support (2),
- en particulier de telle sorte que, en présence d'une pression suffisante, un corps de contact associé du corps de support (2), par exemple un os, est frotté sur la nanostructure (40), et non pas la nanostructure (40) sur le corps de contact quand le corps de support (2) soumis à la pression glisse sur le corps de contact avec le composé en couches (60),
- de préférence le corps de support (2) étant un implant (1), et le composé en couches (60) produisant une action bactéricide et/ou un effet antiadhésif sur la couche la plus extérieure de l'implant (1) qui est constituée conjointement par la nanostructure (40).
